# EUROPEAN PATENT SPECIFICATION

(11) **EP 3 340 869 B1**
(45) Date of publication and mention of the grant of the patent: **01.07.2020**
(21) Application number: 16756860.9
(22) Date of filing: 09.08.2016
(51) Int. Cl.: A61B 5/0215, A61B 5/00

(54) **PRESSURE SENSING GUIDEWIRES**
DRUCKMESSUNGSFÜHRUNGSDRÄHTE
FILS-GUIDES DE DÉTECTION DE PRESSION

(30) Priority: 28.08.2015 US 201562211644 P
(43) Date of publication of application: 04.07.2018
(73) Proprietor: Boston Scientific Scimed Inc., Maple Grove, Minnesota 55311 (US)
(72) Inventor: RADMAN, Lloyd, Blaine, Minnesota 55434 (US); SHIREMAN, Brice Lee, Maple Grove, Minnesota 55311 (US); REYNOLDS, Brian R., Ramsey, Minnesota 55303 (US); MCGOWAN, Roger W., Otsego, Minnesota 55362 (US)
(74) Representative: Vossius & Partner Patentanwälte Rechtsanwälte mbB
(86) International application number: PCT/US2016/046178
(87) International publication number: WO 2017/039979

(56) References cited:
- US-A- 5 166 990
- US-A1- 2014 066 789
- US-A1- 2014 350 414
- ALISON COWLEY ET AL: "A Healthy Future: Platinum in Medical Applications", PLATINIUM METALS REVIEW., vol. 55, no. 2, 1 April 2011 (2011-04-01), pages 98-107, XP055550735, LONDON, GB ISSN: 0032-1400, DOI: 10.1595/147106711X566816
- YAMAGUCHI ET AL: "Safety and Feasibility of an Intravascular Optical Coherence Tomography Image Wire System in the Clinical Setting", AMERICAN JOURNAL OF CARDIOLOGY, CAHNERS PUBLISHING CO., NEWTON, MA, US, vol. 101, no. 5, 10 January 2008 (2008-01-10), pages 562-567, XP022497482, ISSN: 0002-9149, DOI: 10.1016/J.AMJCARD.2007.09.116

## Description

### CROSS-REFERENCE TO RELATED APPLICATIONS

This application claims the benefit of priority to U.S. Provisional Application No. 62/211,644, filed August 28, 2015.

### TECHNICAL FIELD

The present disclosure pertains to medical devices, and methods for manufacturing medical devices. More particularly, the present disclosure pertains to blood pressure sensing guidewires and methods for using pressure sensing guidewires.

### BACKGROUND

A wide variety of intracorporeal medical devices have been developed for medical use, for example, intravascular use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

Patent publications US2014/350414 (A1), US2014/066789 (A1), US5166990 (A), as well as publications ALISON COWLEY ET AL: "A Healthy Future: Platinum in Medical Applications",PLATINIUM METALS REVIEW., vol. 55, no. 2, 1 April 2011 (2011-04-01), and YAMAGUCHI ET AL: "Safety and Feasibility of an Intravascular Optical Coherence Tomography Image Wire System in the Clinical Setting",AMERICAN JOURNAL OF CARDIOLOGY, CAHNERS PUBLISHING CO., NEWTON, MA, US, vol. 101, no. 5, 10 January 2008 (2008-01-10), pages 562-567, are pertinent to understanding the background of the present invention.

### BRIEF SUMMARY

This disclosure provides design, material, manufacturing method, and use alternatives for medical devices. An example medical device for measuring blood pressure is disclosed. The medical device comprises:
a proximal shaft including a proximal lumen extending through the proximal shaft;
a distal member defining a distal lumen extending through the distal member, the distal lumen aligned with the proximal lumen and including a distal lumen inner surface;
a distal cap secured to the distal member, the distal cap including a distal cap inner surface defining a void space;
an optical pressure sensor disposed within the void space within the distal cap; and
a fiber optic cable coupled to the optical pressure sensor and extending proximally through the distal lumen and the proximal lumen, the fiber optic cable having an outer surface;
wherein a tolerance between the outer surface of the fiber optic cable and the inner surface of the distal lumen inner surface limits relative radial movement of the optical pressure sensor relative to the distal cap inner surface such that the optical pressure sensor is constrained from contacting the distal cap inner surface.

According to the invention, the tolerance between the outer surface of the fiber optic cable and the inner surface of the distal lumen inner surface is the range of about 0.005 mm to about 0.05 mm

Alternatively or additionally to any of the embodiments above, a minimum spacing between the optical pressure sensor and the distal cap inner surface further protects the optical pressure sensor from contacting the distal cap inner surface.

Alternatively or additionally to any of the embodiments above, the spacing between the optical pressure sensor and the distal cap inner surface ranges from about 0.005 mm to about 0.1 mm.

Alternatively or additionally to any of the embodiments above, the medical device further comprises one or more apertures formed in the distal cap to permit fluid to enter the void space.

Alternatively or additionally to any of the embodiments above, the distal cap comprises a thin-walled distal cap in order to maximize the void space within the distal cap.

Alternatively or additionally to any of the embodiments above, the distal member comprises a slotted tube.

Alternatively or additionally to any of the embodiments above, the distal cap has an inner diameter that is about equal to an outer diameter of the slotted tube.

Alternatively or additionally to any of the embodiments above, the distal member further comprises a coil disposed outside the slotted tube, and the distal cap has an outer diameter that is less than an outer diameter of the coil.

A medical device for measuring blood pressure is disclosed. The medical device comprises:
a proximal shaft;
a slotted tube secured relative to the proximal shaft and defining a distal lumen extending through the slotted tube;
a coil disposed over the slotted tube;
a distal cap secured relative to the slotted tube and the coil, the distal cap defining a void space;
an optical pressure sensor disposed within the void space within the distal cap; and
a fiber optic cable coupled to the optical pressure sensor and extending proximally through the distal lumen;
wherein the distal lumen is dimensioned to minimize radial movement of the optical pressure sensor relative to the distal cap inner surface; and
wherein the distal cap has an inner diameter that is dimensioned to protect the optical pressure sensor from contacting the distal cap inner surface.

Alternatively or additionally to any of the embodiments above, the medical device further comprising one or more apertures formed in the distal cap to permit fluid to enter the void space.

Alternatively or additionally to any of the embodiments above, the proximal shaft includes a distal region having a pocket formed therein, and the slotted tube extends proximally into the pocket.

Alternatively or additionally to any of the embodiments above, the pocket includes a shoulder region distal of a bottom of the pocket, and the coil extends to the shoulder region.

Alternatively or additionally to any of the embodiments above, the proximal shaft includes a narrowed portion that extends into the distal member, and the narrowed portion forms the slotted tube.

Alternatively or additionally to any of the embodiments above, a transition from the proximal shaft to the narrowed portion of the proximal shaft forms a shoulder region, and the coil terminates at the shoulder region.

Alternatively or additionally to any of the embodiments above, a distal end of the coil is located proximal of a distal end of the slotted tube.

Alternatively or additionally to any of the embodiments above, a proximal end of the distal cap extends proximally over the distal end of the slotted tube and abuts the distal end of the coil.

Alternatively or additionally to any of the embodiments above, the medical device further comprises a collar securing the slotted tube to the proximal shaft.

A medical device for measuring blood pressure is disclosed. The medical device comprises:
a proximal shaft having a distal region;
a distal member having a distal end and a proximal region, the proximal region of the distal member coupled to the distal region of the proximal shaft and extending distally therefrom;
a sensor housing defined proximate the proximal region of the distal member;
an optical pressure sensor disposed within the sensor housing; and
a fiber optic cable coupled to the optical pressure sensor and extending proximally therefrom;
wherein the distal member comprises a cut tube.

Alternatively or additionally to any of the embodiments above, the sensor housing comprises an enlarged inner diameter portion of the proximal shaft.

Alternatively or additionally to any of the embodiments above, the distal member comprises a spiral cut tube.

Alternatively or additionally to any of the embodiments above, the medical device further comprises a shaping ribbon disposed within the distal member and extending a length of a spiral cut of the spiral cut tube.

Alternatively or additionally to any of the embodiments above, the medical device further comprises a radiopaque coil disposed within the spiral cut tube.

Alternatively or additionally to any of the embodiments above, the sensor housing comprises an enlarged inner diameter portion of the distal region of the proximal shaft.

Alternatively or additionally to any of the embodiments above, the cut tube extends distally from the sensor housing.

Alternatively or additionally to any of the embodiments above, the cut tube comprises a plurality of angled cuts.

Alternatively or additionally to any of the embodiments above, the medical device further comprises a shaping ribbon extending distally from the cut tube.

Alternatively or additionally to any of the embodiments above, the medical device further comprises a coil exterior to the cut tube and extending distally to an atraumatic tip.

Alternatively or additionally to any of the embodiments above, the shaping ribbon extends distally to the atraumatic tip.

Alternatively or additionally to any of the embodiments above, the coil is configured to permit fluid to flow between a position exterior to the coil and a position interior to the coil.

Alternatively or additionally to any of the embodiments above, the medical device further comprises a fluid port defined proximal of the sensor housing.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The disclosure may be more completely understood in consideration of the following detailed description in connection with the accompanying drawings, in which:
Figure 1 is a schematic view of a system including a cross-sectional view of an example pressure sensing guidewire;
Figure 2 is a partial cross-sectional side view of a portion of an example medical device;
Figure 3 is a partial cross-sectional view of an example medical device disposed at a first position adjacent to an intravascular occlusion;
Figure 4 is a partial cross-sectional view of an example medical device disposed at a second position adjacent to an intravascular occlusion;
Figures 5 through 7 are partial cross-sectional views of example medical devices;
Figures 8 and 9 are partial cross-sectional views of example medical devices;
Figures 10 through 20 are partial cross-sectional views of example medical devices; and
Figures 21 through 28 are partial cross-sectional views of example medical devices.

While the disclosure is amenable to various modifications and alternative forms, specifics thereof have been shown by way of example in the drawings and will be described in detail. It should be understood, however, that the intention is not to limit the invention to the particular embodiments described. On the contrary, the intention is to cover all modifications, equivalents, and alternatives falling within the scope of the disclosure.

### DETAILED DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary. The scope of protection is defined in the appended claims.

The following detailed description should be read with reference to the drawings in which similar elements in different drawings are numbered the same. The drawings, which are not necessarily to scale, depict illustrative embodiments and are not intended to limit the scope of the invention.

During some medical interventions, it may be desirable to measure and/or monitor the blood pressure within a blood vessel. For example, some medical devices may include pressure sensors that allow a clinician to monitor blood pressure. Such devices may be useful in determining fractional flow reserve (FFR), which may be understood as the ratio of the pressure after a stenosis relative to the pressure before the stenosis (and/or the aortic pressure).

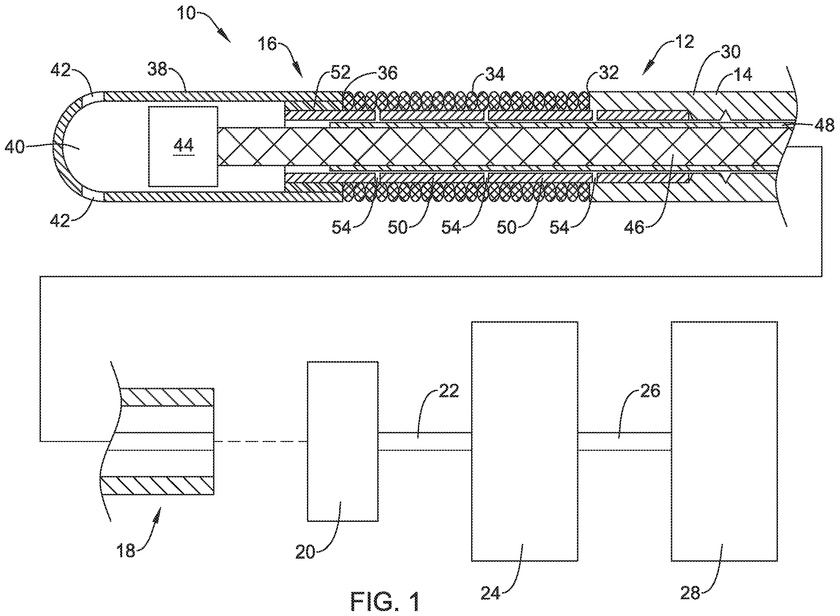

Figure 1 is a schematic view of an example system 10 for obtaining pressure measurements within a patient's anatomy. In some examples, as illustrated, the system 10 includes a pressure sensing guidewire 12 having a guidewire shaft 14 that extends from a distal member 16 to a proximal region 18. At the proximal region 18, the pressure sensing guidewire 12 may be configured to be attached to a connector or handle member 20. The handle 20 may include a suitable connector for a cable 22 to be attached to the handle 20 and extend to another suitable device such as an interferometer or signal conditioner 24. The signal conditioner 24 may include a light source and may be configured to process optical signals received at the signal conditioner 24. Another cable 26 may, in some embodiments, extend from the signal conditioner 24 to a suitable output device such as a display device 28, which may be configured to display information provided by the signal conditioner 24.

The display device 28 may represent the ratio textually, graphically, or pictorially. A clinician may utilize the readings from the display device 28 to tailor an intervention to the needs of the patient or otherwise advance the goals of the intervention. These are just examples. It will be appreciated that other devices and/or arrangements may be utilized with the pressure sensing guidewire 12.

The guidewire shaft 14 includes a first tubular member 30 that terminates at a tubular member distal end 32. In some embodiments, the distal member 16 includes a coil structure 34 that extends distally to a coil structure distal end 36, although a coil structure 34 is not included in all embodiments. In some cases, a distal cap 38 abuts the coil structure distal end 36 and completes the distal portion of the pressure sensing guidewire 12. The distal cap 38 defines a void space 40. One or more apertures 42 extend through a wall forming the distal cap 38, thereby providing fluid communication between the void space 40 and the environment immediately outside of the pressure sensing guidewire 12.

A pressure sensor 44 is disposed within the void space 40. By virtue of the aforementioned fluid communication afforded by the one or more apertures 42, the pressure sensor 44 is configured to obtain pressure measurements within the environment immediately outside of the pressure sensing guidewire 12. While the pressure sensor 44 is shown schematically in Figure 1, it can be appreciated that the structural form and/or type of the pressure sensor 44 may vary. For example, the pressure sensor 44 may include a semiconductor (e.g., silicon wafer) pressure sensor, piezoelectric pressure sensor, a fiber optic or optical pressure sensor, a Fabry-Perot type pressure sensor, an ultrasound transducer and/or ultrasound pressure sensor, a magnetic pressure sensor, a solid-state pressure sensor, or the like, or any other suitable pressure sensor.

A clinician may use the pressure sensing guidewire 12 to measure or calculate FFR (e.g., the pressure after an intravascular lesion relative to the pressure before the lesion). This may include taking an initial pressure reading before, or upstream, of the lesion and then a comparative reading after, or downstream, of the lesion. This may also include monitoring the pressure while advancing the pressure sensing guidewire 12 through a blood vessel until a pressure differential or drop in pressure is observed, indicating that the pressure sensing guidewire 12 has reached and/or is partially past the lesion as well as monitoring increases in pressure during and/or following a treatment intervention. In some embodiments, a second pressure measuring device may be used to measure pressure at another intravascular location and this pressure may be utilized in the calculation of FFR or otherwise used as part of the intervention.

In some embodiments, as illustrated, a fiber optic cable 46 may be operably connected to the pressure sensor 44 and extends proximally therefrom. In some embodiments, fiber optic cable 46 is a glass fiber optic cable and/or a polymer fiber optic cable and may extend a length of the guidewire shaft 14. In some embodiments, the fiber optic cable 46 may include a distal section that is polymeric fiber optic cable and a proximal section that is glass fiber optic cable. In some embodiments, it is contemplated that the fiber optic cable 46 may be a glass fiber optic cable over substantially all of its length. In some embodiments, at least a portion of the fiber optic cable 46 may include a protective coating 48 extending a length of the fiber optic cable 46.

In some embodiments, as illustrated, the pressure sensing guidewire 12 may include a second tubular member 50. The second tubular member 50, if included, may be joined to the distal cap 38 via a connection schematically illustrated as connection 52. The connection 52 may represent adhesive, solder, a weld, or any other suitable connection mechanism. The second tubular member 50 forms part of the distal member 16 and in some embodiments may, in combination with the coil structure 34, contribute to a shapeability of the distal member 16. In some embodiments, the distal member 16 may be bent into a desired shape prior to being advanced through the vasculature. To aid in flexibility, in some embodiments the second tubular member 50 includes several slots 54. While three slots 54 are illustrated, it will be appreciated that the second tubular member 50 may include any number of slots 54. The relative size, number and spacing of the slots 54 along the second tubular member 50 may be varied in order to achieve a desired level of flexibility and shapeability.

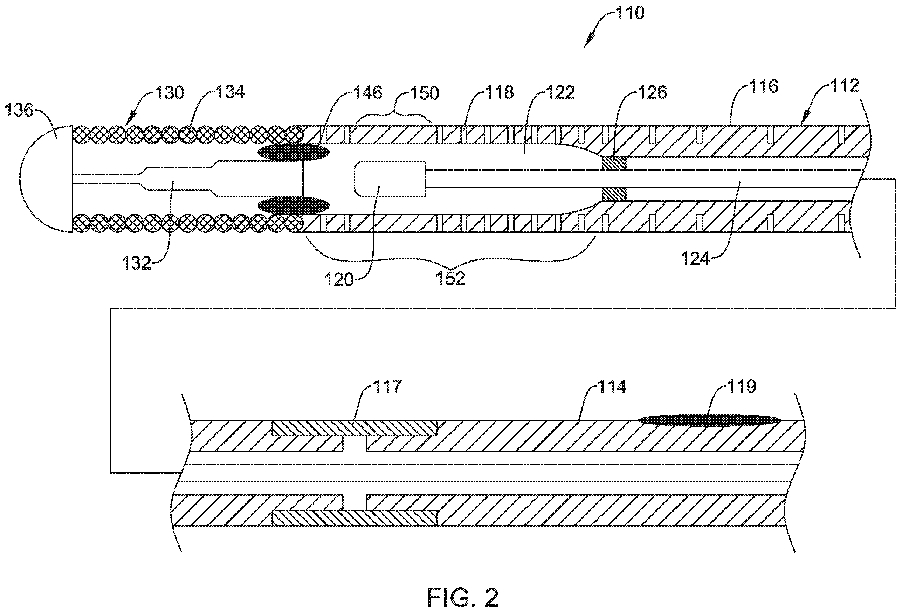

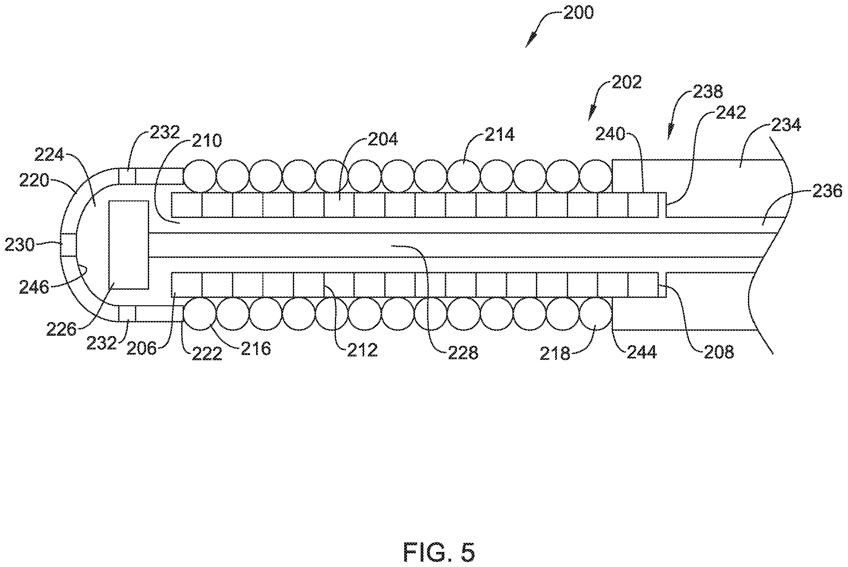

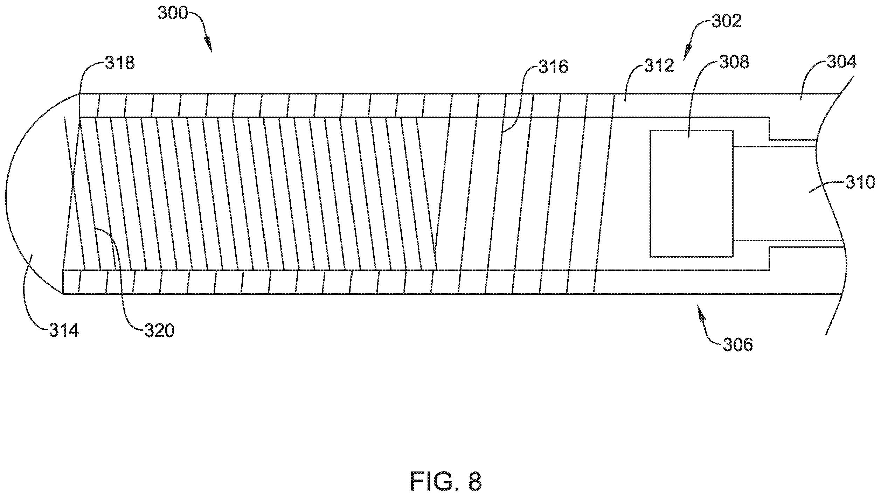

Figure 1 provides an illustrative but non-limiting view at the pressure sensing guidewire 12 that is configured to provide the pressure sensor 44 on a relatively distal position within the distal cap 38. Figures 5-7 provide additional views of various distal tip constructions for a pressure sensing guidewire such as the pressure sensing guidewire 12. In some embodiments, a medical device that is configured to sense blood pressure may instead have a pressure sensor that is still disposed within a distal portion of the medical device yet be positioned more proximally in order to afford a shapeable tip such as a spring tip or a polymer tip that forms a distal end of the medical device that is configured to sense blood pressure, such as a pressure sensing guidewire. Figures 8-28 provide additional views of various distal tip constructions that may be used as part of the pressure sensing guidewire shown in Figure 2.

Figure 2 illustrates a portion of an example medical device 110. In this example, medical device 110 is a blood pressure sensing guidewire 110. However, this is not intended to be limiting as other medical devices are contemplated including, for example, catheters, shafts, leads, wires, or the like. Many constructional features of the pressure sensing guidewire 110 may be similar to those shown in the pressure sensing guidewire 12 (Figure 1), particularly in more proximal portions of the guidewires. The pressure sensing guidewire 110 may include a tubular member or shaft 112. The shaft 112 may include a proximal portion 114 and a distal portion 116. The materials for the proximal portion 114 and the distal portion 116 may vary and may include those materials disclosed herein. For example, the distal portion 116 may include a nickel-cobalt-chromium-molybdenum alloy (e.g., MP35-N) and the proximal portion 114 may include stainless steel. These are just examples. Other materials may also be utilized.

In some embodiments, the proximal portion 114 and the distal portion 116 may be formed from the same monolith of material. In other words, the proximal portion 114 and the distal portion 116 may be portions of the same tube defining the shaft 112. In other embodiments, the proximal portion 114 and the distal portion 116 may be separate tubular members that are joined together. For example, a section of the outer surface of portions 114/116 may be removed and a sleeve 117 may be disposed over the removed sections to join the portions 114/116. Alternatively, the sleeve 117 may be simply disposed over the portions 114/116. Other bonds may also be used including welds, thermal bonds, adhesive bonds, or the like. If utilized, the sleeve 117 used to join the proximal portion 114 with the distal portion 116 may include a material that desirably bonds with both the proximal portion 114 and the distal portion 116. For example, the sleeve 117 may include a nickel-chromium-molybdenum alloy (e.g., INCONEL).

A plurality of slots 118 may be formed in the shaft 112. In at least some embodiments, the slots 118 are formed in the distal portion 116. In at least some embodiments, the proximal portion 114 lacks slots 118. However, the proximal portion 114 may include slots 118. The slots 118 may be desirable for a number of reasons. For example, the slots 118 may provide a desirable level of flexibility to the shaft 112 (e.g., along the distal portion 116) while also allowing suitable transmission of torque. The slots 118 may be arranged/distributed along the distal portion 116 in a suitable manner including any of those arrangements disclosed herein. For example, the slots 118 may be arranged as opposing pairs of slots 118 that are distributed along the length of the distal portion 116. In some embodiments, adjacent pairs of slots 118 may have a substantially constant spacing relative to one another. Alternatively, the spacing between adjacent pairs may vary. For example, more distal regions of the distal portion 116 may have a decreased spacing (and/or increased slot density), which may provide increased flexibility. In other embodiments, more distal regions of the distal portion 116 may have an increased spacing (and/or decreased slot density). These are just examples. Other arrangements are contemplated.

A pressure sensor 120 may be disposed within the shaft 112 (e.g., within a lumen 122 of the shaft 112). While the pressure sensor 120 is shown schematically in Figure 2, it can be appreciated that the structural form and/or type of the pressure sensor 120 may vary, similar to the pressure sensor 44 shown in Figure 1. For example, the pressure sensor 120 may include a semiconductor (e.g., silicon wafer) pressure sensor, piezoelectric pressure sensor, a fiber optic or optical pressure sensor, a Fabry-Perot type pressure sensor, an ultrasound transducer and/or ultrasound pressure sensor, a magnetic pressure sensor, a solid-state pressure sensor, or the like, or any other suitable pressure sensor. In some cases, the sensor 120 may be a different type of sensor, such as a temperature sensor.

As indicated above, the pressure sensor 120 may include an optical pressure sensor. In at least some of these embodiments, an optical fiber or fiber optic cable 124 (e.g., a multimode fiber optic) may be attached to the pressure sensor 120 and may extend proximally therefrom. An attachment member 126 may attach the optical fiber 124 to the shaft 112. The attachment member 126 may be circumferentially disposed about and attached to the optical fiber 124 and may be secured to the inner surface of the shaft 112 (e.g., the distal portion 116). In at least some embodiments, the attachment member 126 is proximally spaced from pressure sensor 120. Other arrangements are contemplated.

In at least some embodiments, the distal portion 116 may include a region with a thinned wall and/or an increased inner diameter that defines a housing region 152. In general, the housing region 152 is the region of the distal portion 116 that ultimately "houses" the pressure sensor (e.g., the pressure sensor 120). By virtue of having a portion of the inner wall of the shaft 112 being removed at the housing region 152, additional space may be created or otherwise defined that can accommodate the sensor 120.

In at least some embodiments, it may be desirable for the pressure sensor 120 to have reduced exposure along its side surfaces to fluid pressure (e.g., from the blood). Accordingly, it may be desirable to position the pressure sensor 120 along a landing region 150 defined along the housing region 152. The landing region 150 may be substantially free of slots 118 so that the side surfaces of the pressure sensor 120 have a reduced likelihood of being deformed due to fluid pressures at these locations. Distal of the landing region 150, the housing region 152 may include slots 118 that provide fluid access to the pressure sensor 120.

Moreover, one or more of the slots 118 may define a fluid pathway that allows blood (and/or a body fluid) to flow from a position along the exterior or outer surface of the guidewire 110 (and/or the shaft 112), through the slots 118, and into a lumen of the shaft 112, where the blood can come into contact with the pressure sensor 120. Because of this, no additional side openings/holes (e.g., other than one or more slots 118, a single slot 118 extending through the wall of the shaft 112, and/or a dedicated pressure port or opening) may be necessary in the shaft 112 for pressure measurement. This may also allow the length of the distal portion 116 to be shorter than typical sensor mounts or hypotubes that would need to have a length sufficient for a suitable opening/hole (e.g., a suitable "large" opening/hole) to be formed therein that provides fluid access to the sensor 120.

A tip member 130 may be coupled to distal portion 116. Tip member 130 may include a shaping member 132 and a spring or coil member 134. A distal tip 136 may be attached to shaping member 132 and/or spring 134. In at least some embodiments, distal tip 136 may take the form of a solder ball tip. Tip member 130 may be joined to distal portion 116 of shaft 112 with a bonding member 146 such as a weld. Subsequent drawings will illustrate other tip members that may be used in combination with a pressure sensing guidewire.

The shaft 112 may include a hydrophilic coating 119. In some embodiments, the hydrophilic coating 119 may extend along substantially the full length of the shaft 112. In other embodiments, one or more discrete sections of the shaft 112 may include the hydrophilic coating 119.

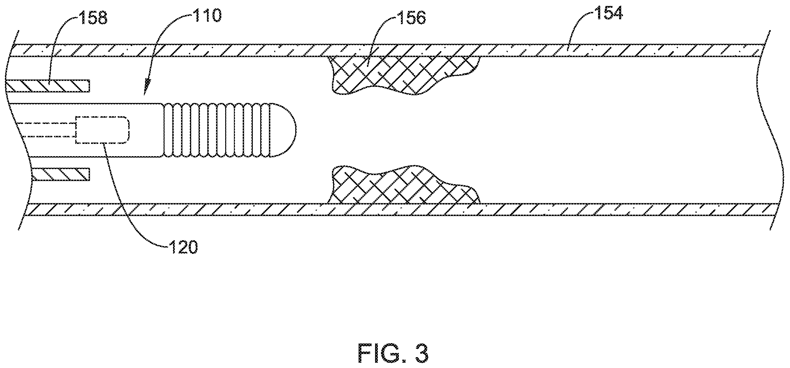

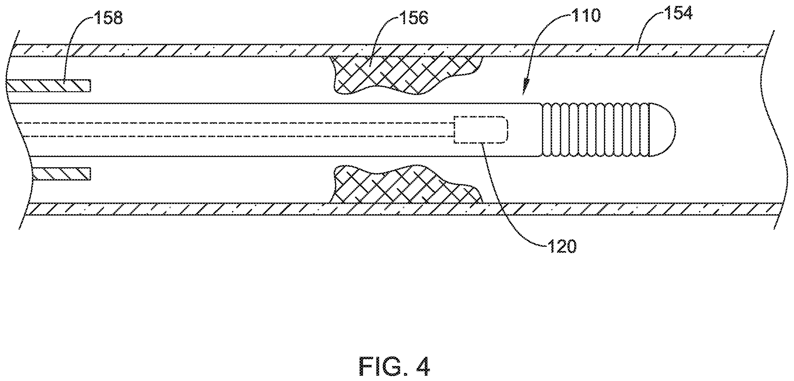

In use, a clinician may use the guidewire 110 to measure and/or calculate FFR (e.g., the pressure after an intravascular occlusion relative to the pressure before the occlusion and/or the aortic pressure). Measuring and/or calculating FFR may include measuring the aortic pressure in a patient. This may include advancing the guidewire 110 through a blood vessel or body lumen 154 to a position that is proximal or upstream of an occlusion 156 as shown in Figure 3. For example, the guidewire 110 may be advanced through a guide catheter 158 to a position where at least a portion of the sensor 120 is disposed distal of the distal end of the guide catheter 158 and measuring the pressure within the body lumen 154. This pressure may be characterized as an initial pressure. In some embodiments, the aortic pressure may also be measured by another device (e.g., a pressure sensing guidewire, catheter, or the like). The initial pressure may be equalized with the aortic pressure. For example, the initial pressure measured by the guidewire 110 may be set to be the same as the measured aortic pressure. The guidewire 110 may be further advanced to a position distal or downstream of the occlusion 156 as shown in Figure 4 and the pressure within the body lumen 154 may be measured. This pressure may be characterized as the downstream or distal pressure. The distal pressure and the aortic pressure may be used to calculate FFR.

It can be appreciated that an FFR system that utilizes an optical pressure sensor in a pressure sensing guidewire may be navigated through the tortuous anatomy. This may include crossing relatively tight bends in the vasculature. Because of this, and for other reasons, it may be desirable of pressure sensing guidewire to be relatively flexible, for example adjacent to the distal end. It can be appreciated that in relatively flexible guidewires, bending the guidewire could result in contact between an inner surface of the guidewire and, for example, the pressure sensor. Such contact could lead to alterations and/or deformations of the pressure sensor, potentially leading to pressure reading offsets. Accordingly, disclosed herein are pressure-sensing guidewires that may include structural features that may help to reduce contact between the pressure sensor and the inner surface of the guidewire and, therefore, help to reduce the possibility of pressure reading offsets.

Figures 5-7 provide additional examples of pressure sensing guidewires that are configured with a pressure sensor disposed near the distal end of the pressure sensing guidewire, such as within a distal cap forming the distal end of the pressure sensing guidewire. It will be appreciated, for example, that the distal members shown in Figures 5-7 may replace at least part of the distal region 16 of the pressure sensing guidewire 12 shown in Figure 1. In some embodiments, it will also be appreciated that a pressure sensing guidewire may combine or otherwise incorporate elements or features that are shown on multiple guidewires.

Figure 5 provides a cross-sectional view of a distal member 200 that forms part of a pressure sensing guidewire 202. In some embodiments, as illustrated, the distal member 200 includes a slotted tube 204 that extends from a distal end 206 to a proximal end 208, and that defines a lumen 210 extending through the slotted tube 204. The slotted tube 204 includes a plurality of slots 212 that are cut at least partially into the slotted tube 204. In some cases, the slots 212 help to improve the flexibility of the slotted tube 204. While the slotted tube 204 is illustrated as having a constant outer diameter, in some cases the slotted tube 204 may be tapered, for example, to provide additional flexibility.

A coil 214 may be disposed over the slotted tube 204. The coil 214 extends from a distal end 216 to a proximal end 218. While not illustrated, in some cases at least part of the coil 214 may include a polymeric coating or sleeve in order to reduce or eliminate fluid travel between adjacent windings of the coil 214. In some cases, there is no polymeric coating or sleeve. While the coil 214 is illustrated as being tightly wound, with little or no space between adjacent windings, it will be appreciated that in some instances the coil 214 may have an open winding pattern, or a varying winding pattern in order to provide the coil 214 with a desired level of flexibility. In some cases, it is contemplated that at least some of the adjacent windings may be secured together, such as with an adhesive or spot weld in order to provide a desired level of torqueability. In some cases, the coil 214 may instead be disposed interior to the slotted tube 204.

A distal cap 220 extends distally from the slotted tube 204 and/or the coil 214. In some cases, the distal cap 220 has a proximal end 222 that abuts the distal end 216 of the coil 214. In some instances, as illustrated, the distal end 206 of the slotted tube 204 extends further distally than the distal end 216 of the coil 214. In some cases, however, the distal end 206 of the slotted tube 204 may be aligned with the distal end 216 of the coil 214. In some instances, the distal end 216 of the coil 214 may extend further distally than the distal end 206 of the slotted tube 204. The distal cap 220 may be adhesively secured to the slotted tube 204 and/or the coil 214, or may be welded or soldered in position.

The distal cap 220 defines a void space 224 that may be sized to accommodate a pressure sensor 226. The pressure sensor 226 may be any desired type of pressure sensor, and in some cases may be an optical pressure sensor. A fiber optic cable 228 extends proximally from the pressure sensor 226 and extends through the lumen 210 defined by the slotted tube 204. As illustrated, the distal cap 220 includes an end aperture 230 that is located at or near a distal end of the distal cap 220 and several side apertures 232 that are disposed proximally of the end aperture 230. In some cases, the side apertures 232 may be disposed proximally of the pressure sensor 226 such that fluid flow entering one of the end aperture 230 or the side apertures 232 will flow past the pressure sensor 226 and exit through the other of the end aperture 230 or the side apertures 232. It will be appreciated that in an arterial approach, blood flow will be from proximal to distal while in a vascular approach, blood flow will be from distal to proximal. In some cases, permitting blood flow past the pressure sensor 226 may be beneficial in eliminating bubbles and other debris.

The pressure sensing guidewire 202 includes a proximal shaft 234 defining a proximal lumen 236 extending therethrough. In some cases, the proximal shaft 234 has a distal end 238 including a pocket 240 that is defined within the distal end 238 as well as a shoulder 244 adjacent the pocket 240. The pocket 240 includes a pocket bottom 242. In some embodiments, as illustrated, the slotted tube 204 extends proximally into the pocket 240 such that the proximal end 208 of the slotted tube 204 abuts the pocket bottom 240. In some cases, as shown, the coil 214 extends proximally such that the proximal end 218 of the coil 214 abuts the shoulder 244. It will be appreciated that this connection between the distal member 200 and the proximal shaft 234 is merely illustrative.

In some embodiments, a tolerance between an outer diameter of the fiber optic cable 228 and an inner diameter of the lumen 210 (defined by the slotted tube 204) may help to limit radial movement of the pressure sensor 226 relative to an interior surface 246 of the distal cap 220 sufficiently such that the pressure sensor 226 is less likely to contact the interior surface 246 of the distal cap 220. According to the invention, this tolerance is in the range of about 0.005 millimeters (mm) to about 0.05 mm. In some instances, this tolerance may extend into the proximal lumen 236 while in some cases the proximal lumen 236 may be sized to provide a greater tolerance in order to facilitate assembly.

In some cases, a minimum spacing between the pressure sensor 226 and the interior surface 246 of the distal cap 220 also helps to minimize possible contact between the pressure sensor 226 and the interior surface 246 of the distal cap 220. According to an exemplary construction, this spacing may be in the range of about 0.005 mm to about 0.1 mm, or about 0.01 mm to about 0.08 mm, measured as the shortest distance between the pressure sensor 226 and the interior surface 246 of the distal cap 220. It will be appreciated that in some cases, the spacing may be achieved by changing the outer dimensions of the pressure sensor 226. In some cases, the distal cap 220 may be selected to have a relatively thin wall thickness in order to provide the desired spacing. In some cases, a combination of the tolerance between an outer diameter of the fiber optic cable 228 and an inner diameter of the lumen 210 (defined by the slotted tube 204) and the spacing between the pressure sensor 226 may help prevent incidental contact between the pressure sensor 226 and the interior surface 246 of the distal cap 220.

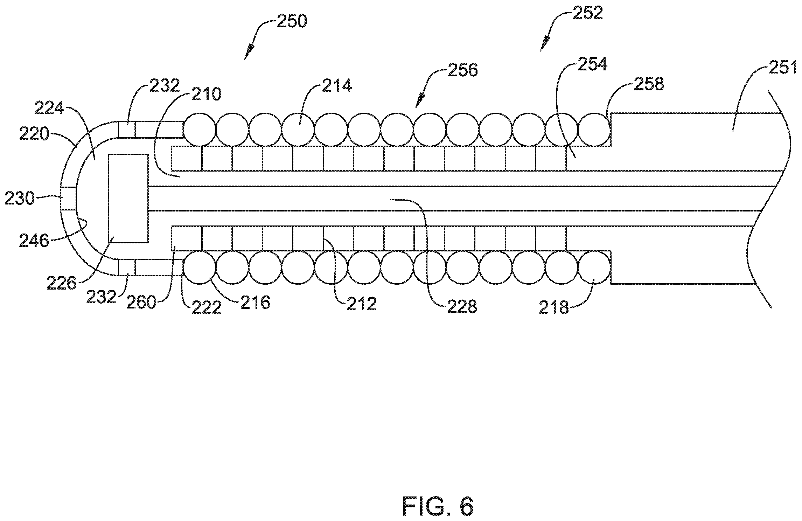

Figure 6 provides a cross-sectional view of a distal member 250 that forms a distal portion of a pressure sensing guidewire 252. In some embodiments, as illustrated, the pressure sensing guidewire 252 includes a proximal shaft 251 having a narrowed distal portion 254 that defines a slotted tube 256. In some cases, the narrowed distal portion 254 is integrally formed with the proximal shaft 251, and may for example represent a portion of the proximal shaft 251 that has been milled down or otherwise processed to have a narrower profile, and to include a plurality of slots 212 formed therein in order to improve flexibility. In some cases, a shoulder 258 is formed adjacent the narrowed distal portion 254. The slotted tube 256 extends distally to a distal end 260 and defines the lumen 210 extending therethrough. While the slotted tube 256 is illustrated as having a constant outer diameter, in some cases the slotted tube 256 may be tapered, for example, to provide additional flexibility.

A coil 214 may be disposed over the slotted tube 256. The coil 214 extends from a distal end 216 to a proximal end 218. While not illustrated, in some cases at least part of the coil 214 may include a polymeric coating or sleeve in order to reduce or eliminate fluid travel between adjacent windings of the coil 214. In some cases, there is no polymeric coating or sleeve. While the coil 214 is illustrated as being tightly wound, with little or no space between adjacent windings, it will be appreciated that in some instances the coil 214 may have an open winding pattern, or a varying winding pattern in order to provide the coil 214 with a desired level of flexibility. In some cases, it is contemplated that at least some of the adjacent windings may be secured together, such as with an adhesive or spot weld in order to provide a desired level of torqueability. In some cases, the coil 214 may instead be disposed interior to the slotted tube 256. In some cases, the proximal end 218 of the coil 214 is disposed adjacent to and optionally secured to the shoulder 258 formed within the proximal shaft 251.

A distal cap 220 extends distally from the slotted tube 256 and/or the coil 214. In some cases, the distal cap 220 has a proximal end 222 that abuts the distal end 216 of the coil 214. In some instances, as illustrated, the distal end 260 of the slotted tube 256 extends further distally than the distal end 216 of the coil 214. In some cases, however, the distal end 260 of the slotted tube 204 may be aligned with the distal end 216 of the coil 214. In some instances, the distal end 216 of the coil 214 may extend further distally than the distal end 260 of the slotted tube 256. The distal cap 220 may be adhesively secured to the slotted tube 256 and/or the coil 214, or may be welded or soldered in position.

The distal cap 220 defines a void space 224 that may be sized to accommodate a pressure sensor 226. The pressure sensor 226 may be any desired type of pressure sensor, and in some cases may be an optical pressure sensor. A fiber optic cable 228 extends proximally from the pressure sensor 226 and extends through the lumen 210 defined by the slotted tube 256. As illustrated, the distal cap 220 includes an end aperture 230 that is located at or near a distal end of the distal cap 220 and several side apertures 232 that are disposed proximally of the end aperture 230. In some cases, the side apertures 232 may be disposed proximally of the pressure sensor 226 such that fluid flow entering one of the end aperture 230 or the side apertures 232 will flow past the pressure sensor 226 and exit through the other of the end aperture 230 or the side apertures 232. It will be appreciated that in an arterial approach, blood flow will be from proximal to distal while in a vascular approach, blood flow will be from distal to proximal. In some cases, permitting blood flow past the pressure sensor 226 may be beneficial in eliminating bubbles and other debris.

In some embodiments, as discussed for example with respect to Figure 5, a tolerance between an outer diameter of the fiber optic cable 228 and an inner diameter of the lumen 210 (defined by the slotted tube 256) may help to limit radial movement of the pressure sensor 226 relative to an interior surface 246 of the distal cap 220 sufficiently such that the pressure sensor 226 is less likely to contact the interior surface 246 of the distal cap 220. In some cases, a spacing between the pressure sensor 226 and the interior surface 246 of the distal cap 220 also helps to minimize possible contact between the pressure sensor 226 and the interior surface 246 of the distal cap 220.

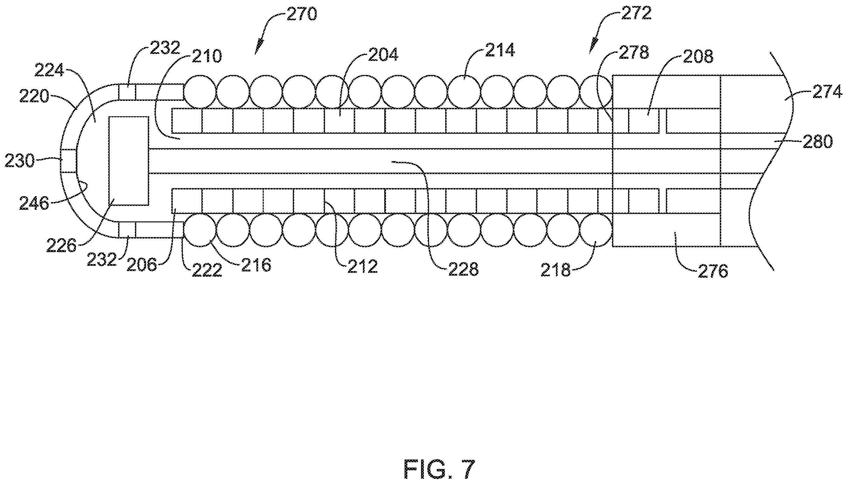

Figure 7 provides a cross-sectional view of a distal member 270 that forms a distal portion of a pressure sensing guidewire 272. In some embodiments, as illustrated, the distal member 270 includes a slotted tube 204 that extends from a distal end 206 to a proximal end 208, and that defines a lumen 210 extending through the slotted tube 204. The slotted tube 204 includes a plurality of slots 212 that are cut at least partially into the slotted tube 204. In some cases, the slots 212 help to improve the flexibility of the slotted tube 204. While the slotted tube 204 is illustrated as having a constant outer diameter, in some cases the slotted tube 204 may be tapered, for example, to provide additional flexibility.

A coil 214 may be disposed over the slotted tube 204. The coil 214 extends from a distal end 216 to a proximal end 218. While not illustrated, in some cases at least part of the coil 214 may include a polymeric coating or sleeve in order to reduce or eliminate fluid travel between adjacent windings of the coil 214. In some cases, there is no polymeric coating or sleeve. While the coil 214 is illustrated as being tightly wound, with little or no space between adjacent windings, it will be appreciated that in some instances the coil 214 may have an open winding pattern, or a varying winding pattern in order to provide the coil 214 with a desired level of flexibility. In some cases, it is contemplated that at least some of the adjacent windings may be secured together, such as with an adhesive or spot weld in order to provide a desired level of torqueability. In some cases, the coil 214 may instead be disposed interior to the slotted tube 204.

A distal cap 220 extends distally from the slotted tube 204 and/or the coil 214. In some cases, the distal cap 220 has a proximal end 222 that abuts the distal end 216 of the coil 214. In some instances, as illustrated, the distal end 206 of the slotted tube 204 extends further distally than the distal end 216 of the coil 214. In some cases, however, the distal end 206 of the slotted tube 204 may be aligned with the distal end 216 of the coil 214. In some instances, the distal end 216 of the coil 214 may extend further distally than the distal end 206 of the slotted tube 204. The distal cap 220 may be adhesively secured to the slotted tube 204 and/or the coil 214, or may be welded or soldered in position.

The distal cap 220 defines a void space 224 that may be sized to accommodate a pressure sensor 226. The pressure sensor 226 may be any desired type of pressure sensor, and in some cases may be an optical pressure sensor. A fiber optic cable 228 extends proximally from the pressure sensor 226 and extends through the lumen 210 defined by the slotted tube 204. As illustrated, the distal cap 220 includes an end aperture 230 that is located at or near a distal end of the distal cap 220 and several side apertures 232 that are disposed proximally of the end aperture 230. In some cases, the side apertures 232 may be disposed proximally of the pressure sensor 226 such that fluid flow entering one of the end aperture 230 or the side apertures 232 will flow past the pressure sensor 226 and exit through the other of the end aperture 230 or the side apertures 232. It will be appreciated that in an arterial approach, blood flow will be from proximal to distal while in a vascular approach, blood flow will be from distal to proximal. In some cases, permitting blood flow past the pressure sensor 226 may be beneficial in eliminating bubbles and other debris.

The pressure sensing guidewire 272 includes a proximal shaft 274 defining a proximal lumen 280 extending therethrough. In some cases, the proximal shaft 274 may include a collar 276 that fits about and secures the proximal end 208 of the slotted tube 204. In some cases, the collar 276 has a distal surface 278 against which the proximal end 218 of the coil 214 may rest against and/or be secured to.

In some embodiments, as discussed for example with respect to Figure 5, a tolerance between an outer diameter of the fiber optic cable 228 and an inner diameter of the lumen 210 (defined by the slotted tube 204) may help to limit radial movement of the pressure sensor 226 relative to an interior surface 246 of the distal cap 220 sufficiently such that the pressure sensor 226 is less likely to contact the interior surface 246 of the distal cap 220. In some cases, a spacing between the pressure sensor 226 and the interior surface 246 of the distal cap 220 also helps to minimize possible contact between the pressure sensor 226 and the interior surface 246 of the distal cap 220.

Figures 5-7 provided illustrative but non-limiting examples of distal members that are configured to place the pressure sensor at or near the distal tip of the guidewire, such as the guidewire 10 shown in Figure 1. Figures 8-9 provide particular examples of distal members that place the pressure sensor at a more proximal location, such as the guidewire 110 shown in Figure 2. It will be appreciated, for example, that the distal members shown in Figures 8-9 may replace at least part of the distal region 116 of the pressure sensing guidewire 112 shown in Figure 2. In some embodiments, it will also be appreciated that a pressure sensing guidewire may combine or otherwise incorporate elements or features that are shown on multiple guidewires.

Figure 8 provides a cross-sectional view of a distal member 300 that forms a distal portion of a pressure sensing guidewire 302. A proximal shaft 304 terminates in a sensor housing 306 that may be considered as being disposed between the proximal shaft 304 and the distal member 300. In some cases, the sensor housing 306 represents a widened inner diameter portion of the pressure sensing guidewire 302. A pressure sensor 308 is disposed within the sensor housing 306. While the pressure sensor 308 may be any of a variety of different pressure sensors, in some cases the pressure sensor 308 is an optical pressure sensor and thus is coupled to a fiber optic cable 310 that extends proximally from the pressure sensor 308.

In some embodiments, the distal member 300 may be considered as being largely hollow. The pressure sensing guidewire 302 may include a tubular distal shaft 312 that extends distally to an atraumatic tip 314. In some instances, as illustrated, the tubular distal shaft 312 may include a spiral cut 316 that extends from a position near the sensor housing 306 to a distal end 318 of the tubular distal shaft 312. It will be appreciated that in some cases this spiral cut 316 may be dimensioned to permit blood flowing outside of the distal member 300 to pass into the interior of the distal member 300 and thus reach the sensor housing 306 and the pressure sensor 308. While not illustrated, in some cases the sensor housing 306 may also include one or more apertures to permit blood to flow in or out of the sensor housing 306, depending on whether an arterial or vascular approach is taken.

In some embodiments, the distal member 300 may further include a radiopaque coil 320, such as but not limited to a platinum coil. In some cases, this may improve visibility of the pressure sensing guidewire 302 during procedures. In some instances, the distal member 300 may instead include a polymeric coating that includes a radiopaque loading, for example.

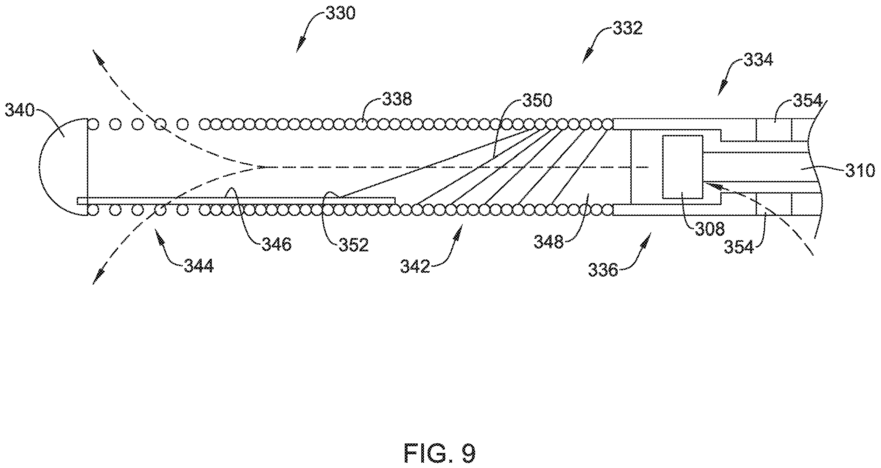

Figure 9 provides a cross-sectional view of a distal member 330 that forms a distal portion of a pressure sensing guidewire 332. A proximal shaft 334 terminates in a sensor housing 336 that may be considered as being disposed between the proximal shaft 334 and the distal member 330. In some cases, the sensor housing 336 represents a widened inner diameter portion of the pressure sensing guidewire 332. A pressure sensor 308 is disposed within the sensor housing 336. While the pressure sensor 308 may be any of a variety of different pressure sensors, in some cases the pressure sensor 308 is an optical pressure sensor and thus is coupled to a fiber optic cable 310 that extends proximally from the pressure sensor 308.

In some embodiments, the distal member 330 may be considered as being largely hollow. The pressure sensing guidewire 332 may include a coil 338 that extends from the sensor housing 336 to an atraumatic tip 340. In some cases, the coil 338 may include a tightly wound proximal portion 342 and a loosely wound distal portion 344. In some cases, the loosely wound distal portion 344 has a spacing between adjacent windings that is sufficient to permit blood to flow between an exterior of the distal member 330 to an interior of the distal member 330. It will be appreciated that the relative direction of flow, i.e., whether blood enters or exits through the loosely wound distal portion 344 may depend on whether an arterial or vascular approach is taken. In some cases, the sensor housing 336 may include one or more apertures 354 to permit blood flow therethrough.

In some embodiments, the distal member 330 may include a shaping ribbon 346 that extends through the loosely wound distal portion 344 and that extends distally to the atraumatic tip 340. In some instances, a slotted tube 348 is disposed inside of the coil 338 and extends distally from the sensor housing 336. The slotted tube 348 may include a spiral cut 350 having an increasing cut angle approaching a distal end 352 of the slotted tube 348. In some cases, the shaping ribbon 346 extends distally from a position at or near the distal end 352 of the slotted tube 348.

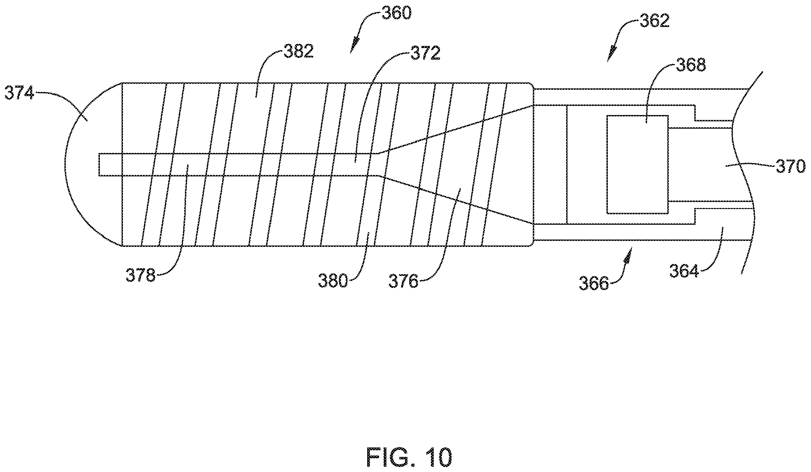

Figures 10 through 20 provide additional examples of distal members that place the pressure sensor at a more proximal location, such as the guidewire 110 shown in Figure 2. It will be appreciated, for example, that the distal members shown in Figures 10-28 may replace at least part of the distal region 116 of the pressure sensing guidewire 112 shown in Figure 2. In some embodiments, it will also be appreciated that a pressure sensing guidewire may combine or otherwise incorporate elements or features that are shown on multiple guidewires.

Figure 10 provides a cross-sectional view of a distal member 360 that forms a distal portion of a pressure sensing guidewire 362. A proximal shaft 364 terminates in a sensor housing 366 that may be considered as being disposed between the proximal shaft 364 and the distal member 360. In some cases, the sensor housing 366 represents a widened inner diameter portion of the pressure sensing guidewire 362. A pressure sensor 368 is disposed within the sensor housing 366. While the pressure sensor 368 may be any of a variety of different pressure sensors, in some cases the pressure sensor 368 is an optical pressure sensor and thus is coupled to a fiber optic cable 370 that extends proximally from the pressure sensor 368. While not expressly illustrated, the distal member 360 and/or the sensor housing 366 may include one or more apertures to permit blood to flow in and/or out of the sensor housing 366.

A tapered core 372 extends distally from the sensor housing 366 and may extend distally to an atraumatic tip 374. In some cases, as illustrated, the tapered core 372 includes a tapered portion 376 and a straight portion 378, but in other embodiments the tapered core 372 may include one or more tapered portions and one or more straight portions, as desired. In some cases, the distal member 360 includes a coil 380 that in some cases may be at least partially encased within a polymer covering 382. In some cases, the polymeric covering 382 may include a radiopaque loading, but this is not required in all embodiments.

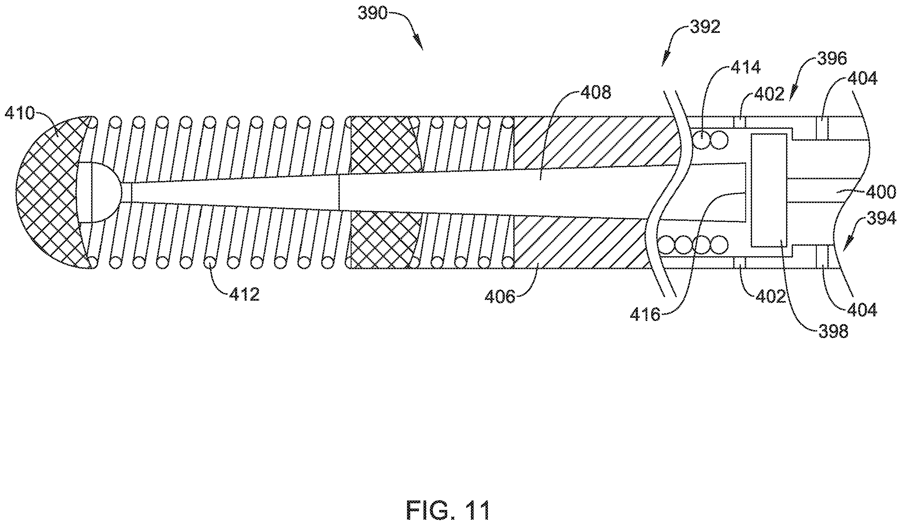

Figure 11 provides a cross-sectional view of a distal member 390 that forms a distal portion of a pressure sensing guidewire 392. A proximal shaft 394 terminates in a sensor housing 396 that may be considered as being disposed between the proximal shaft 394 and the distal member 390. In some cases, the sensor housing 396 represents a widened inner diameter portion of the pressure sensing guidewire 392. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures 402 disposed on a distal side of the pressure sensor 398 and one or more apertures 404 disposed on a proximal side of the pressure sensor 398 to permit blood to flow in and/or out of the sensor housing 396. As noted above, the relative flow direction will depend in part upon whether an arterial or vascular approach is taken.

The distal member 390 includes a shaft 406 that may be polymeric or metallic and that extends distally from the sensor housing 396. In some embodiments, a tapered core 408 extends through the shaft 406 and to an atraumatic tip 410. In some cases, a coil 412 may be disposed over the tapered core 408 and may extend from the shaft 406 to the atraumatic tip 410. In some cases, a centering coil 414 may be disposed relative a proximal end 416 of the tapered core 408.

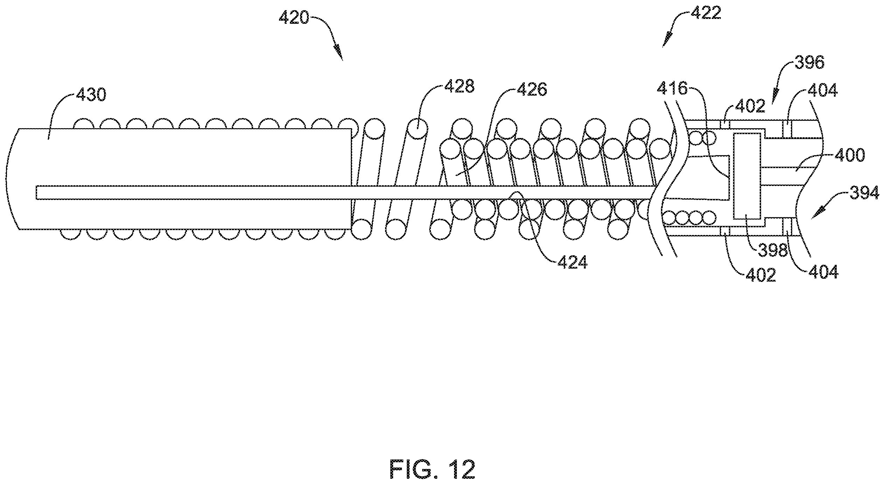

Figure 12 provides a cross-sectional view of a distal member 420 that forms a distal portion of a pressure sensing guidewire 422. A proximal shaft 394 terminates in a sensor housing 396 that may be considered as being disposed between the proximal shaft 394 and the distal member 420. In some cases, the sensor housing 396 represents a widened inner diameter portion of the pressure sensing guidewire 422. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures 402 disposed on a distal side of the pressure sensor 398 and one or more apertures 404 disposed on a proximal side of the pressure sensor 398 to permit blood to flow in and/or out of the sensor housing 396. As noted above, the relative flow direction will depend in part upon whether an arterial or vascular approach is taken. The distal member 420 extends distally from the sensor housing 396. In some embodiments, a tapered core 424 extends distally from the sensor housing 396. An inner coil 426 is disposed over the tapered core 424 and in some cases extends proximally into the sensor housing 396. An outer coil 428 may be disposed over the inner coil 426 and may extend distally to a distal structure 430 that may, for example, include one or more of solder, a weld or adhesive.

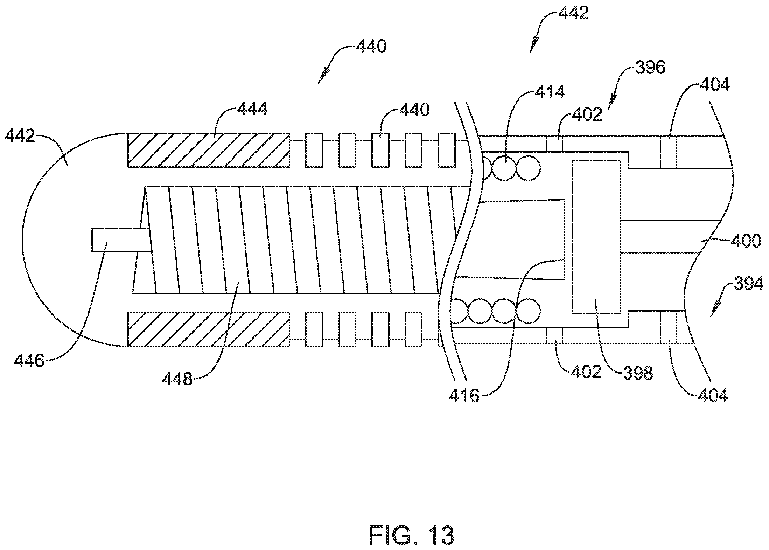

Figure 13 provides a cross-sectional view of a distal member 440 that forms a distal portion of a pressure sensing guidewire 442. A proximal shaft 394 terminates in a sensor housing 396 that may be considered as being disposed between the proximal shaft 394 and the distal member 440. In some cases, the sensor housing 396 represents a widened inner diameter portion of the pressure sensing guidewire 442. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures 402 disposed on a distal side of the pressure sensor 398 and one or more apertures 404 disposed on a proximal side of the pressure sensor 398 to permit blood to flow in and/or out of the sensor housing 396. As noted above, the relative flow direction will depend in part upon whether an arterial or vascular approach is taken. The distal member 440 includes a slotted tube 441 that extends distally to an atraumatic tip 442. In some cases, an adhesive or other polymeric assembly 444 secures the atraumatic tip 442 to the slotted tube 441. In some cases, a core member 446 extends within the slotted tube 441, with a spiral cut tube 448 disposed over the core member 446 but within the slotted tube 441.

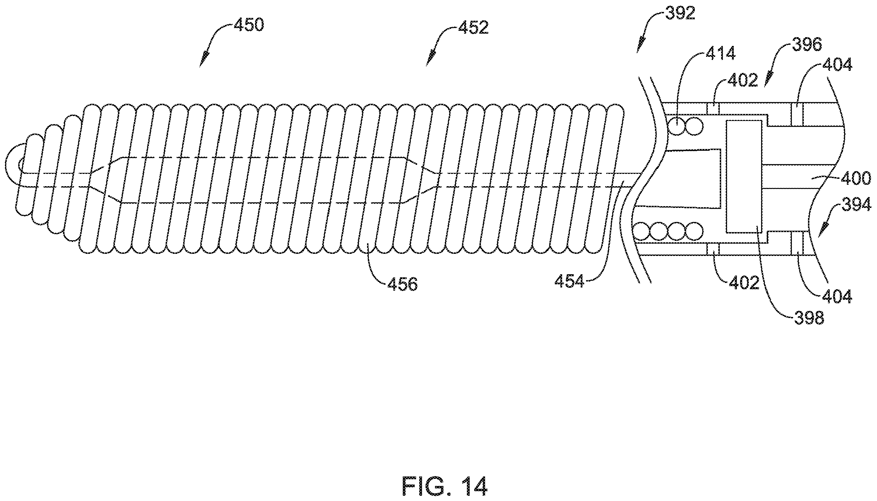

Figure 14 provides a cross-sectional view of a distal member 450 that forms a distal portion of a pressure sensing guidewire 452. A proximal shaft 394 terminates in a sensor housing 396 that may be considered as being disposed between the proximal shaft 394 and the distal member 450. In some cases, the sensor housing 396 represents a widened inner diameter portion of the pressure sensing guidewire 452. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures 402 disposed on a distal side of the pressure sensor 398 and one or more apertures 404 disposed on a proximal side of the pressure sensor 398 to permit blood to flow in and/or out of the sensor housing 396. As noted above, the relative flow direction will depend in part upon whether an arterial or vascular approach is taken. A core 454 extends distally from the sensor housing 396. In some cases, the core 454 extends back over itself and is wound to form a coil 456 that extends over the core 454. In some cases, the coil 456 is separately formed and then attached to the core 454.

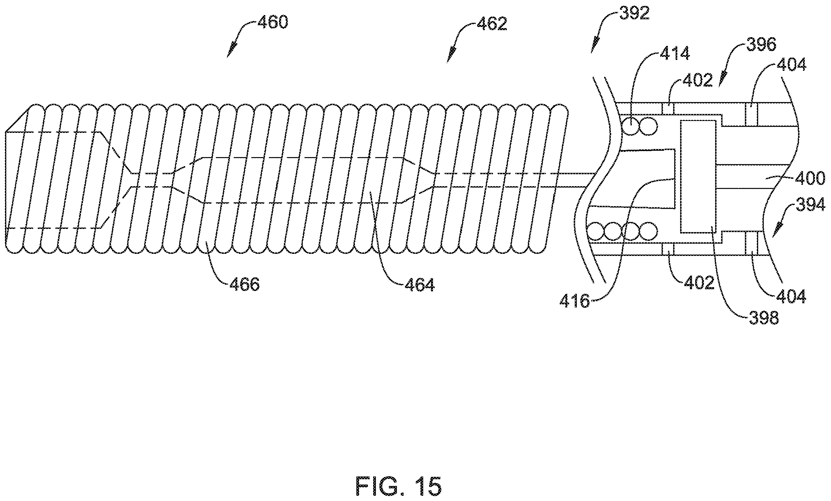

Figure 15 provides a cross-sectional view of a distal member 460 that forms a distal portion of a pressure sensing guidewire 462. A proximal shaft 394 terminates in a sensor housing 396 that may be considered as being disposed between the proximal shaft 394 and the distal member 460. In some cases, the sensor housing 396 represents a widened inner diameter portion of the pressure sensing guidewire 462. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures 402 disposed on a distal side of the pressure sensor 398 and one or more apertures 404 disposed on a proximal side of the pressure sensor 398 to permit blood to flow in and/or out of the sensor housing 396. As noted above, the relative flow direction will depend in part upon whether an arterial or vascular approach is taken. A core 464 that may have several different cross-sectional profiles including a stamped region extends distally from the sensor housing 396. A coil 466 extends over the core 464.

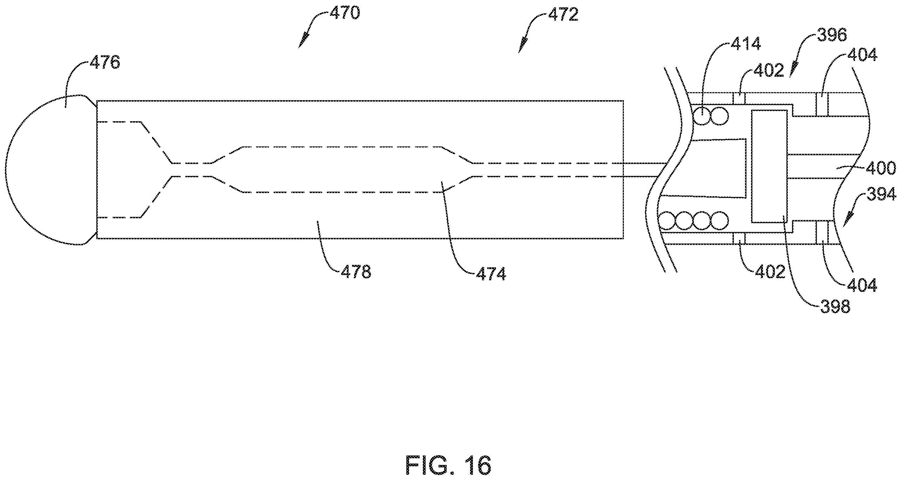

Figure 16 provides a cross-sectional view of a distal member 470 that forms a distal portion of a pressure sensing guidewire 472. A proximal shaft 394 terminates in a sensor housing 396 that may be considered as being disposed between the proximal shaft 394 and the distal member 470. In some cases, the sensor housing 396 represents a widened inner diameter portion of the pressure sensing guidewire 472. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures 402 disposed on a distal side of the pressure sensor 398 and one or more apertures 404 disposed on a proximal side of the pressure sensor 398 to permit blood to flow in and/or out of the sensor housing 396. As noted above, the relative flow direction will depend in part upon whether an arterial or vascular approach is taken. A core 474, which may have a profile similar to that of the core 464 (Figure 15) extends distally from the sensor housing 396 to an atraumatic tip 476. In some cases, a polymeric sheath 478 may encase the core 474.

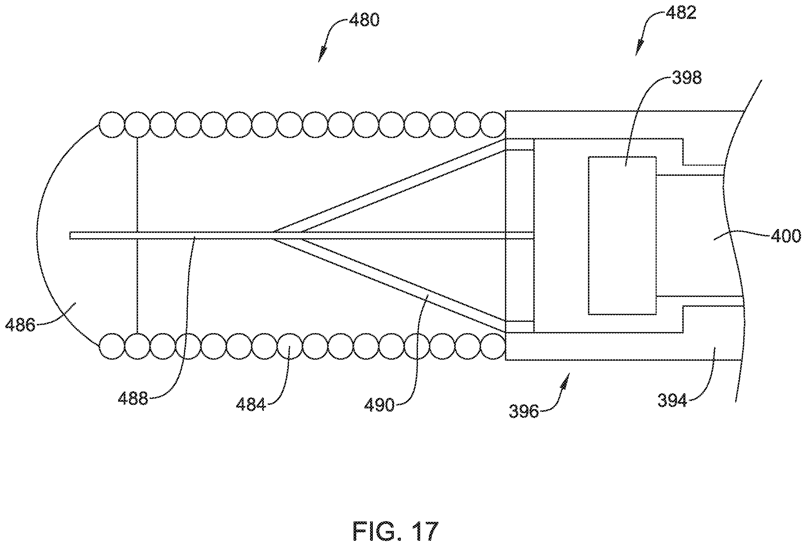

Figure 17 provides a cross-sectional view of a distal member 480 that forms a distal portion of a pressure sensing guidewire 482. A proximal shaft 394 terminates in a sensor housing 396 that may be considered as being disposed between the proximal shaft 394 and the distal member 480. In some cases, the sensor housing 396 represents a widened inner diameter portion of the pressure sensing guidewire 482. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures (not illustrated) to permit blood to flow in and/or out of the sensor housing 396. As noted above, the relative flow direction will depend in part upon whether an arterial or vascular approach is taken. A coil 484 extends distally from the sensor housing 396 to an atraumatic tip 486. A ribbon support 488 extends distally to the atraumatic tip 486 and is secured to a center ring 490 that may, as illustrated, include several additional ribbon supports for stability.

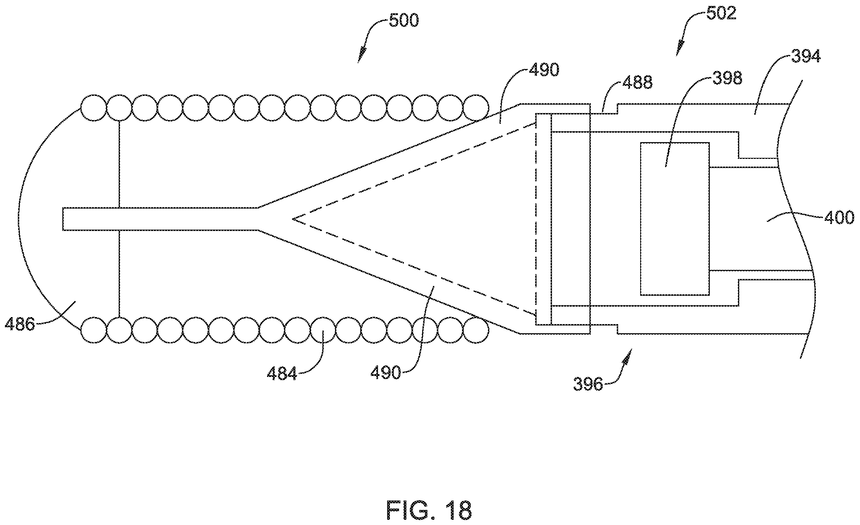

Figure 18 provides a cross-sectional view of a distal member 500 that forms a distal portion of a pressure sensing guidewire 502. A proximal shaft 394 terminates in a sensor housing 396 that may be considered as being disposed between the proximal shaft 394 and the distal member 500. In some cases, the sensor housing 396 represents a widened inner diameter portion of the pressure sensing guidewire 502. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures (not illustrated) to permit blood to flow in and/or out of the sensor housing 396. As noted above, the relative flow direction will depend in part upon whether an arterial or vascular approach is taken. A hollow conical core member 490 extends distally from a narrowed portion 488 of the sensor housing 396 to an atraumatic tip 486. In some cases, a coil 484 extends between the core member 490 and the atraumatic tip 486.

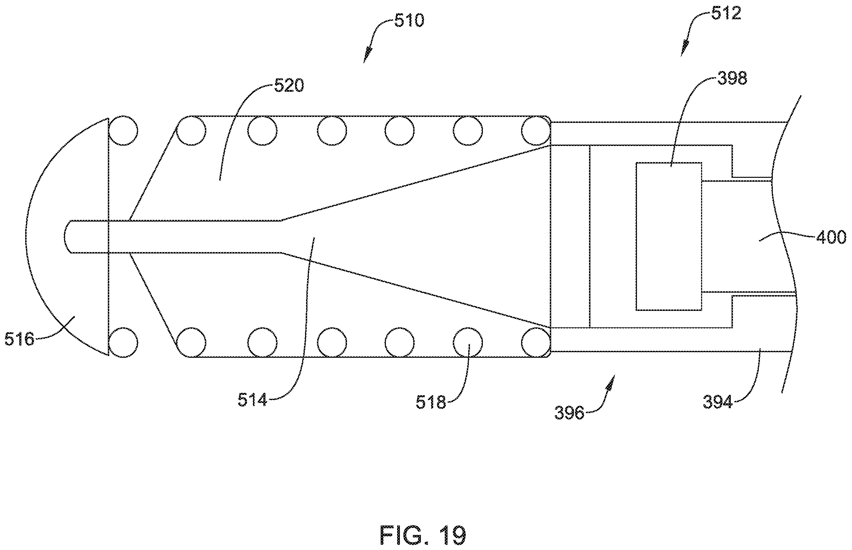

Figure 19 provides a cross-sectional view of a distal member 510 that forms a distal portion of a pressure sensing guidewire 512. A proximal shaft 394 terminates in a sensor housing 396 that may be considered as being disposed between the proximal shaft 394 and the distal member 510. In some cases, the sensor housing 396 represents a widened inner diameter portion of the pressure sensing guidewire 512. A pressure sensor 398 is disposed within the sensor housing 396. While the pressure sensor 398 may be any of a variety of different pressure sensors, in some cases the pressure sensor 398 is an optical pressure sensor and thus is coupled to a fiber optic cable 400 that extends proximally from the pressure sensor 398. The sensor housing 396 may include one or more apertures (not illustrated) to permit blood to flow in and/or out of the sensor housing 396. As noted above, the relative flow direction will depend in part upon whether an arterial or vascular approach is taken. A tapered core member 514 extends distally from the sensor housing 396 to an atraumatic tip 516. In some cases, a coil 518 extends between the sensor housing 396 and the atraumatic tip 516. A polymeric sleeve 520 is disposed over the tapered core member 514 and the coil 518.

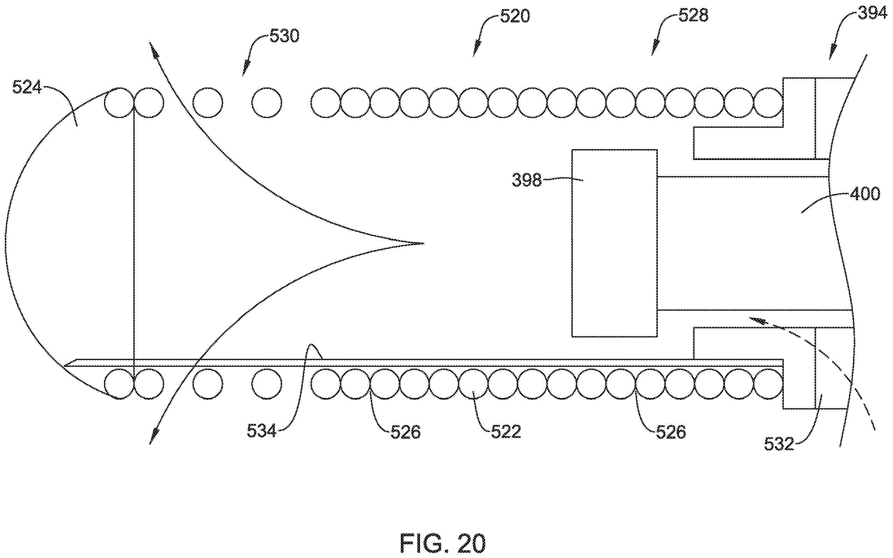

Figure 20 provides a cross-sectional view of a distal member 520 that accommodates the pressure sensor 398 and the fiber optic cable 400 within a proximal portion of the distal member 520. The distal member 520 includes a coil 522 that extends between an atraumatic tip 524 and the proximal shaft 394. In some cases, as illustrated, the coil 522 includes one or more weld spots 526 that can be used to control flexibility of the distal member 520. If greater stiffness is desired, more weld spots 526 may be used. If less stiffness is desired, fewer weld spots 526 may be used. In some cases, the coil 522 includes a proximal portion 528 in which adjacent windings of the coil 522 are close together and a distal portion 520 in which at least some of the adjacent windings of the coil 522 are spaced sufficiently apart to permit blood to flow through. One or more apertures 532 may be disposed proximal of the pressure sensor 398 to permit blood flow. Relative flow direction may be a function of whether an arterial or vascular approach is taken. In some cases, a shaping ribbon 534 extends from the proximal shaft 394 to the atraumatic tip 524.

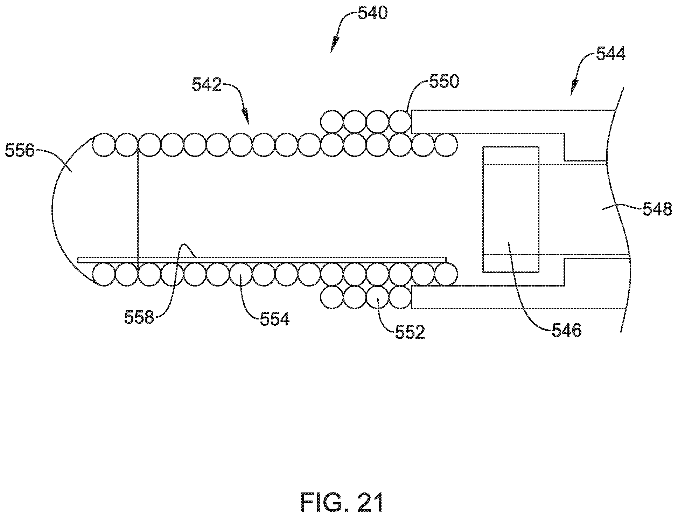

Figures 21 through 28 provide additional examples of distal members having particular spring tip designs disposed distal of the pressure sensor. It will be appreciated, for example, that the distal members shown in Figures 21-28 may replace at least part of the distal region 116 of the pressure sensing guidewire 112 shown in Figure 2. In some embodiments, it will also be appreciated that a pressure sensing guidewire may combine or otherwise incorporate elements or features that are shown on multiple guidewires.

Figure 21 provides a cross-sectional view of a distal member 540 that includes a spring tip 542 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 542 includes a short outer coil 552 extending distally from the distal end 550 and a longer inner coil 554 that extends proximally into the sensor housing 544 and that extends distally to an atraumatic tip 556. In some embodiments, a shaping ribbon 558 extends within the inner coil 554.

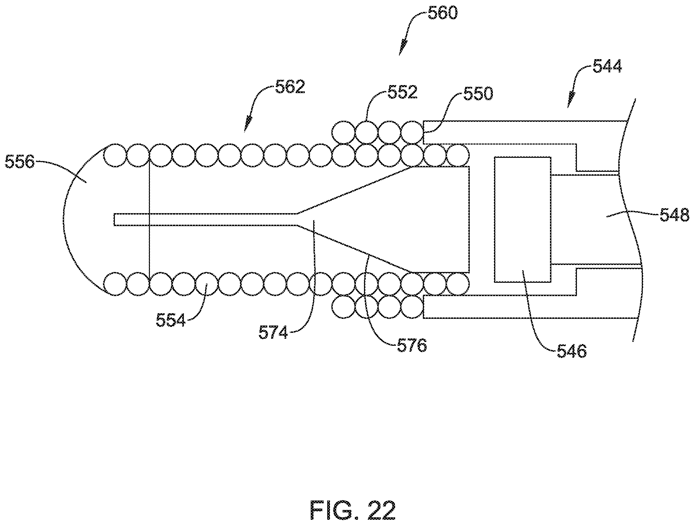

Figure 22 provides a cross-sectional view of a distal member 560 that includes a spring tip 562 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 562 includes a short outer coil 552 extending distally from the distal end 550. In some embodiments, a tapered core 574 extends distally from the sensor housing 544 to the atraumatic tip 556. In some cases, a longer inner coil 554 extends distally from a tapered portion 576 of the tapered core 574 and extends to the atraumatic tip 556.

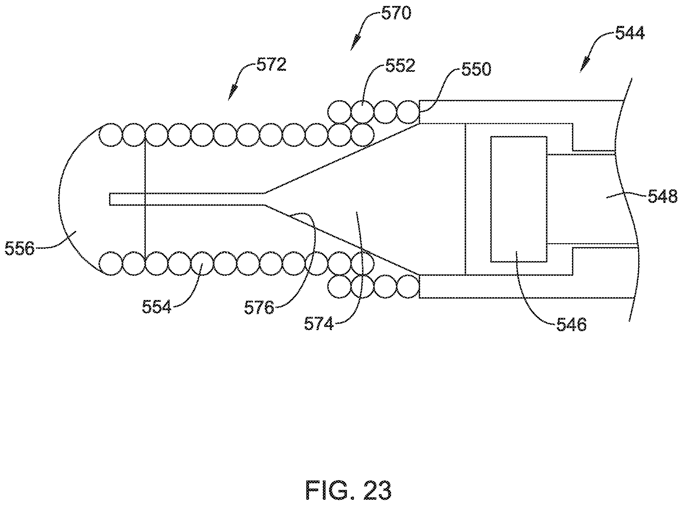

Figure 23 provides a cross-sectional view of a distal member 570 that includes a spring tip 572 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 572 includes a short outer coil 552 extending distally from the distal end 550. In some embodiments, a tapered core 574 extends distally from the sensor housing 544 to the atraumatic tip 556. In some cases, a longer inner coil 554 extends distally from a tapered portion 576 of the tapered core 574 and extends to the atraumatic tip 556.

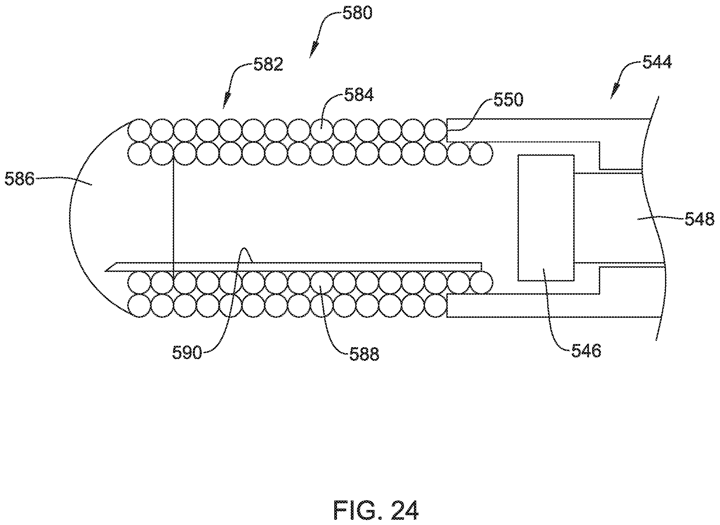

Figure 24 provides a cross-sectional view of a distal member 580 that includes a spring tip 582 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 582 includes an outer coil 584 that extends from the distal end 550 of the sensor housing 544 to an atraumatic tip 586. An inner coil 588 extends within the outer coil 584. In some embodiments, a shaping ribbon 590 extends within the inner coil 588.

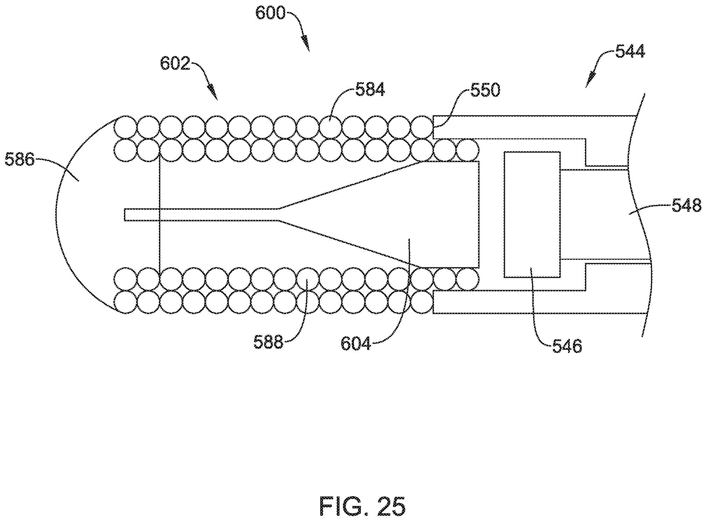

Figure 25 provides a cross-sectional view of a distal member 600 that includes a spring tip 602 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 602 includes an outer coil 584 that extends from the distal end 550 of the sensor housing 544 to an atraumatic tip 586. An inner coil 588 extends within the outer coil 584. In some embodiments, a tapered core 604 extends within the inner coil 588.

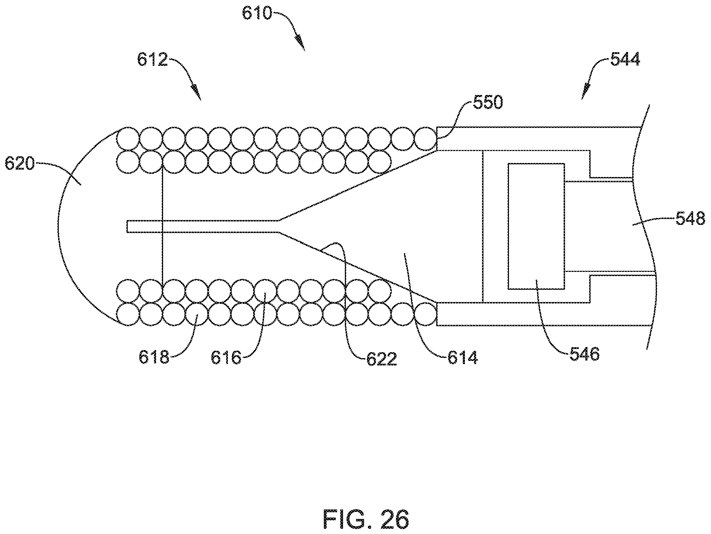

Figure 26 provides a cross-sectional view of a distal member 610 that includes a spring tip 612 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 612 includes a tapered core 614 extending distally to an atraumatic tip 620. An inner coil 616 extends distally from a tapered region 622 of the tapered core 614 and is secured to the atraumatic tip 620. An outer coil 618 extends from the distal end 550 of the sensor housing 544 and is secured to the atraumatic tip 620.

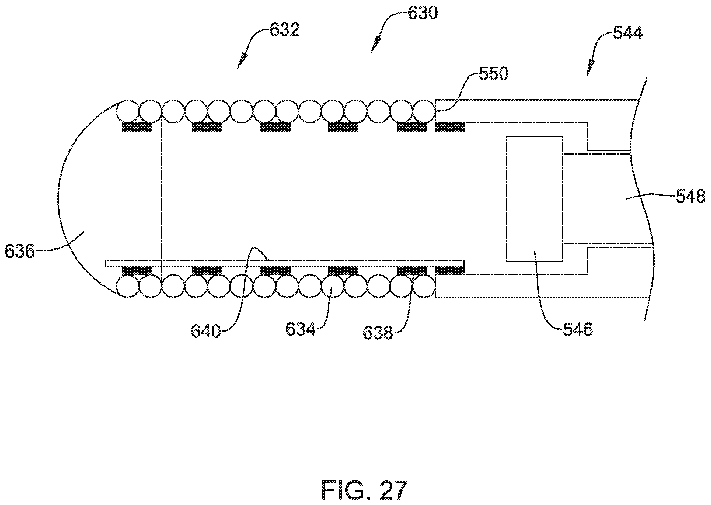

Figure 27 provides a cross-sectional view of a distal member 630 that includes a spring tip 632 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 632 includes a coil 634 extending distally from the distal end 550 of the sensor housing 544 to an atraumatic tip 636. In some cases, a slotted tube 638 extends within the coil 634 and a shaping ribbon 640 may extend within the slotted tube 638.

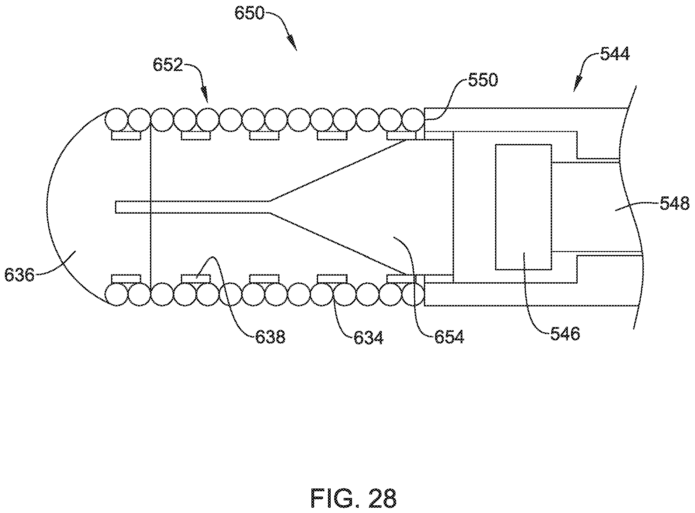

Figure 28 provides a cross-sectional view of a distal member 650 that includes a spring tip 652 extending distally from a sensor housing 544. The sensor housing 544 includes an optical pressure sensor 546 coupled to a fiber optic cable 548 that extends proximally from the optical pressure sensor 546. The sensor housing 544, which may for example be formed as a distal end of a proximal shaft (not illustrated) has a distal end 550. As illustrated, the spring tip 652 includes a coil 634 extending distally from the distal end 550 of the sensor housing 544 to an atraumatic tip 636. In some cases, a slotted tube 638 extends within the coil 634. A tapered core 654 is disposed within the slotted tube 638 and extends distally to the atraumatic tip 636.

The materials that can be used for the various components of the pressure sensing guidewires (and components thereof) may include those commonly associated with medical devices. For simplicity purposes, the following discussion makes reference to shaft 12, 112 and other components of guidewire 10, 110. However, this is not intended to limit the devices and methods described herein, as the discussion may be applied to other tubular members and/or components of tubular members or devices disclosed herein.

Shaft 12, 112 may be made from a metal, metal alloy, polymer (some examples of which are disclosed below), a metal-polymer composite, ceramics, combinations thereof, and the like, or other suitable material. Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; titanium; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear that the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also can be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. In other words, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Some examples of nickel titanium alloys are disclosed in U.S. Patent Nos. 5,238,004 and 6,508,803. Other suitable materials may include ULTANIUM™ (available from Neo-Metrics) and GUM METAL™ (available from Toyota). In some other embodiments, a superelastic alloy, for example a superelastic nitinol can be used to achieve desired properties.

In at least some embodiments, portions or all of shaft 12, 112 may also be doped with, made of, or otherwise include a radiopaque material. Radiopaque materials are understood to be materials capable of producing a relatively bright image on a fluoroscopy screen or another imaging technique during a medical procedure. This relatively bright image aids the user of guidewire 10, 110 in determining its location. Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of guidewire 10, 110 to achieve the same result.

In some embodiments, a degree of Magnetic Resonance Imaging (MRI) compatibility is imparted into guidewire 10, 110. For example, shaft 12, 112 or portions thereof may be made of a material that does not substantially distort the image and create substantial artifacts (i.e., gaps in the image). Certain ferromagnetic materials, for example, may not be suitable because they may create artifacts in an MRI image. Shaft 12, 112, or portions thereof, may also be made from a material that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

A sheath or covering (not shown) may be disposed over portions or all of shaft 12, 112 that may define a generally smooth outer surface for guidewire 10, 110. In other embodiments, however, such a sheath or covering may be absent from a portion of all of guidewire 10, 110, such that shaft 12, 112 may form the outer surface. The sheath may be made from a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN® available from DuPont), polyether block ester, polyurethane (for example, Polyurethane 85A), polypropylene (PP), polyvinylchloride (PVC), polyether-ester (for example, ARNITEL® available from DSM Engineering Plastics), ether or ester based copolymers (for example, butylene/poly(alkylene ether) phthalate and/or other polyester elastomers such as HYTREL® available from DuPont), polyamide (for example, DURETHAN® available from Bayer or CRISTAMID® available from Elf Atochem), elastomeric polyamides, block polyamide/ethers, poly ether block amide (PEBA, for example available under the trade name PEBAX®), ethylene vinyl acetate copolymers (EVA), silicones, polyethylene (PE), Marlex high-density polyethylene, Marlex low-density polyethylene, linear low density polyethylene (for example REXELL®), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR®), polysulfone, nylon, nylon-12 (such as GRILAMID® available from EMS American Grilon), perfluoro(propyl vinyl ether) (PFA), ethylene vinyl alcohol, poly olefin, polystyrene, epoxy, polyvinylidene chloride (PVdC), poly(styrene-*b*-isobutylene-*b*-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

In some embodiments, the exterior surface of the guidewire 10, 110 (including, for example, the exterior surface of shaft 12, 112) may be sandblasted, beadblasted, sodium bicarbonate-blasted, electropolished, etc. In these as well as in some other embodiments, a coating, for example a lubricious, a hydrophilic, a protective, or other type of coating may be applied over portions or all of the sheath, or in embodiments without a sheath over portion of shaft 12, 112, or other portions of guidewire 10, 110. Alternatively, the sheath may comprise a lubricious, hydrophilic, protective, or other type of coating. Hydrophobic coatings such as fluoropolymers provide a dry lubricity which improves guidewire handling and device exchanges. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility. Some other examples of such coatings and materials and methods used to create such coatings can be found in U.S. Patent Nos. 6,139,510 and 5,772,609.

The coating and/or sheath may be formed, for example, by coating, extrusion, co-extrusion, interrupted layer co-extrusion (ILC), or fusing several segments end-to-end. The layer may have a uniform stiffness or a gradual reduction in stiffness from the proximal end to the distal end thereof. The gradual reduction in stiffness may be continuous as by ILC or may be stepped as by fusing together separate extruded tubular segments. The outer layer may be impregnated with a radiopaque filler material to facilitate radiographic visualization. Those skilled in the art will recognize that these materials can vary widely without deviating from the scope of the present disclosure.

Various embodiments of arrangements and configurations of slots are also contemplated that may be used in addition to what is described above or may be used in alternate embodiments. For simplicity purposes, the following disclosure makes reference to guidewire 10, 110, slots 54 and 118, and shaft 12, 112. However, it can be appreciated that these variations may also be utilized for other slots and/or tubular members. In some embodiments, at least some, if not all of slots 54, 118 are disposed at the same or a similar angle with respect to the longitudinal axis of the shaft. As shown, slots 54, 118 can be disposed at an angle that is perpendicular, or substantially perpendicular, and/or can be characterized as being disposed in a plane that is normal to the longitudinal axis of the shaft. However, in other embodiments, slots 54, 118 can be disposed at an angle that is not perpendicular, and/or can be characterized as being disposed in a plane that is not normal to the longitudinal axis of shaft 12, 112. Additionally, a group of one or more slots 54, 118 may be disposed at different angles relative to another group of one or more slots 54, 118. The distribution and/or configuration of slots 54, 118 can also include, to the extent applicable, any of those disclosed in U.S. Pat. Publication No. US 2004/0181174.

Slots 54, 118 may be provided to enhance the flexibility of shaft 12, 112 while still allowing for suitable torque transmission characteristics. Slots 54, 118 may be formed such that one or more rings and/or tube segments interconnected by one or more segments and/or beams that are formed in shaft 12, 112, and such tube segments and beams may include portions of shaft 12, 112 that remain after slots 54 118 are formed in the body of shaft 12, 112. Such an interconnected structure may act to maintain a relatively high degree of torsional stiffness, while maintaining a desired level of lateral flexibility. In some embodiments, some adjacent slots 54, 118 can be formed such that they include portions that overlap with each other about the circumference of shaft 12, 112. In other embodiments, some adjacent slots 54, 118 can be disposed such that they do not necessarily overlap with each other, but are disposed in a pattern that provides the desired degree of lateral flexibility.

Additionally, slots 54, 118 can be arranged along the length of, or about the circumference of, shaft 12, 112 to achieve desired properties. For example, adjacent slots 54, 118, or groups of slots, can be arranged in a symmetrical pattern, such as being disposed essentially equally on opposite sides about the circumference of shaft 12, 112, or can be rotated by an angle relative to each other about the axis of shaft 12, 112. Additionally, adjacent slots 54, 118, or groups of slots, may be equally spaced along the length of shaft 12, 112, or can be arranged in an increasing or decreasing density pattern, or can be arranged in a non-symmetric or irregular pattern. Other characteristics, such as slot size, slot shape, and/or slot angle with respect to the longitudinal axis of shaft 12, 112, can also be varied along the length of shaft 12, 112 in order to vary the flexibility or other properties. In other embodiments, moreover, it is contemplated that the portions of the tubular member, such as a proximal section, or a distal section, or the entire shaft 12, 112, may not include any such slots 54, 118.

As suggested herein, slots 54, 118 may be formed in groups of two, three, four, five, or more slots 54, 118, which may be located at substantially the same location along the axis of shaft 12, 112. Alternatively, a single slot 54, 118 may be disposed at some or all of these locations. Within the groups of slots 54, 118, there may be included slots that are equal in size (i.e., span the same circumferential distance around shaft 12, 112). In some of these as well as other embodiments, at least some slots in a group are unequal in size (i.e., span a different circumferential distance around shaft 12, 112). Longitudinally adjacent groups of slots 54, 118 may have the same or different configurations. For example, some embodiments of shaft 12, 112 include slots 54, 118 that are equal in size in a first group and then unequally sized in an adjacent group. It can be appreciated that in groups that have two slots 54, 118 that are equal in size and are symmetrically disposed around the tube circumference, the centroid of the pair of beams (i.e., the portion of shaft remaining after slots 18 are formed therein) is coincident with the central axis of shaft. Conversely, in groups that have two slots that are unequal in size and whose centroids are directly opposed on the tube circumference, the centroid of the pair of beams can be offset from the central axis of shaft. Some embodiments of shaft include only slot groups with centroids that are coincident with the central axis of the shaft, only slot groups with centroids that are offset from the central axis of the shaft, or slot groups with centroids that are coincident with the central axis of shaft 12, 112 in a first group and offset from the central axis of shaft 12, 112 in another group. The amount of offset may vary depending on the depth (or length) of slots 54, 118 and can include other suitable distances.

Slots 54, 118 can be formed by methods such as micro-machining, saw-cutting (e.g., using a diamond grit embedded semiconductor dicing blade), electron discharge machining, grinding, milling, casting, molding, chemically etching or treating, or other known methods, and the like. In some such embodiments, the structure of the shaft is formed by cutting and/or removing portions of the tube to form slots 54, 118. Some example embodiments of appropriate micromachining methods and other cutting methods, and structures for tubular members including slots and medical devices including tubular members are disclosed in U.S. Pat. Publication Nos. 2003/0069522 and 2004/0181174-A2; and U.S. Pat. Nos. 6,766,720; and 6,579,246. Some example embodiments of etching processes are described in U.S. Pat. No. 5,106,455.

In at least some embodiments, slots 54, 118 may be formed in a tubular member using a laser cutting process. The laser cutting process may include a suitable laser and/or laser cutting apparatus. For example, the laser cutting process may utilize a fiber laser. Utilizing processes like laser cutting may be desirable for a number of reasons. For example, laser cutting processes may allow the shaft to be cut into a number of different cutting patterns in a precisely controlled manner. This may include variations in the slot width, ring width, beam height and/or width, etc. Furthermore, changes to the cutting pattern can be made without the need to replace the cutting instrument (e.g., blade). This may also allow smaller tubes (e.g., having a smaller outer diameter) to be used to form the shaft without being limited by a minimum cutting blade size.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The scope of the disclosure is of course, defined in the wording in which the appended claims are expressed.

## Claims

1. A medical device for measuring blood pressure, comprising:
a proximal shaft (18, 234) including a proximal lumen (236) extending through the proximal shaft;
a distal member (16, 200) defining a distal lumen (210) extending through the distal member, the distal lumen aligned with the proximal lumen and including a distal lumen inner surface;
a distal cap (38, 220) secured to the distal member, the distal cap including a distal cap inner surface (246) defining a void space (40, 224);
an optical pressure sensor (44, 226) disposed within the void space within the distal cap; and
a fiber optic cable (46, 228) coupled to the optical pressure sensor and extending proximally through the distal lumen and the proximal lumen, the fiber optic cable having an outer surface;
wherein a tolerance between the outer surface of the fiber optic cable and the inner surface of the distal lumen inner surface limits relative radial movement of the optical pressure sensor relative to the distal cap inner surface such that the optical pressure sensor is constrained from contacting the distal cap inner surface, **characterized in that**:
the tolerance being in the range of about 0.005 mm to about 0.05 mm.

2. The medical device of claim 1, wherein a spacing between the optical pressure sensor and the distal cap inner surface further protects the optical pressure sensor from contacting the distal cap inner surface.

3. The medical device of any ones of claims 1 or 2, further comprising one or more apertures (42, 230, 232) formed in the distal cap to permit fluid to enter the void space.

4. The medical device of any one of claims 1 to 3, wherein the distal member comprises a slotted tube (204).

5. The medical device of claim 4, wherein the distal cap has an inner diameter that is about equal to an outer diameter of the slotted tube.

6. The medical device of claim 5, wherein the distal member further comprises a coil (214) disposed outside the slotted tube, and the distal cap has an outer diameter that is less than an outer diameter of the coil.

7. The medical device of claim 6, wherein the proximal shaft (234) includes a distal region (238) having a pocket (240) formed therein and the slotted tube extends proximally into the pocket and optionally the pocket includes a shoulder region (244) distal of a bottom (242) of the pocket, with the coil extending to the shoulder region.

8. The medical device of claim 6, wherein the proximal shaft includes a narrowed portion (254) that extends into the distal member and forms the slotted tube and optionally a transition from the proximal shaft to the narrowed portion of the proximal shaft forms a shoulder region (258), with the coil terminating at the shoulder region.

9. The medical device of any one of claims 6 to 8, wherein a distal end of the coil is located proximal of a distal end of the slotted tube and a proximal end of the distal cap extends proximally over the distal end of the slotted tube and abuts the distal end of the coil.

10. The medical device of any one of claims 6 to 9, further comprising a collar (276) securing the slotted tube to the proximal shaft.

11. The medical device of claim 1,
wherein the distal member comprises a cut tube (448).

12. The medical device of claim 11, wherein the distal member comprises a spiral cut tube or a cut tube having a plurality of angled cuts.

13. The medical device of any one of claims 11 or 12, further comprising a shaping ribbon (346).

14. The medical device of any one of claims 11 to 13, wherein the distal member comprises a coil that is configured to permit fluid to flow between a position exterior to the coil and a position interior to the coil and the medical device further comprises a fluid port (354) defined proximal of the void space.

## Patentansprüche

1. Medizinprodukt zur Blutdruckmessung, das aufweist:
einen proximalen Schaft (18, 234) mit einem proximalen Lumen (236), das sich durch den proximalen Schaft erstreckt;
ein distales Teil (16, 200), das ein distales Lumen (210) bildet, das sich durch das distale Teil erstreckt, wobei das distale Lumen mit dem proximalen Lumen ausgerichtet ist und eine Innenfläche des distalen Lumens aufweist;
eine distale Kappe (38, 220), die am distalen Teil befestigt ist, wobei die distale Kappe eine Innenfläche (246) der distalen Kappe aufweist, die einen Hohlraum (40, 224) bildet;
einen optischen Drucksensor (44, 226), der im Hohlraum in der distalen Kappe angeordnet ist; und
ein faseroptisches Kabel (46, 228), das mit dem optischen Drucksensor gekoppelt ist und sich durch das distale Lumen und das proximale Lumen proximal erstreckt, wobei das faseroptische Kabel eine Außenfläche hat;
wobei eine Toleranz zwischen der Außenfläche des faseroptischen Kabels und der Innenfläche der Innenfläche des distalen Lumens relative Radialbewegung des optischen Drucksensors relativ zur Innenfläche der distalen Kappe so begrenzt, dass der optische Drucksensor daran gehindert ist, die Innenfläche der distalen Kappe zu kontaktieren,
**dadurch gekennzeichnet, dass**:
die Toleranz im Bereich von etwa 0,005 mm bis etwa 0,05 mm liegt.

2. Medizinprodukt nach Anspruch 1, wobei ein Abstand zwischen dem optischen Drucksensor und der Innenfläche der distalen Kappe den optischen Drucksensor ferner davor schützt, die Innenfläche der distalen Kappe zu kontaktieren.

3. Medizinprodukt nach Anspruch 1 oder 2, ferner mit einer oder mehreren Öffnungen (42, 230, 232), die in der distalen Kappe gebildet sind, damit Fluid in den Hohlraum eintreten kann.

4. Medizinprodukt nach einem der Ansprüche 1 bis 3, wobei das distale Teil eine Schlitzröhre (204) aufweist.

5. Medizinprodukt nach Anspruch 4, wobei die distale Kappe einen Innendurchmesser hat, der etwa gleich einem Außendurchmesser der Schlitzröhre ist.

6. Medizinprodukt nach Anspruch 5, wobei das distale Teil ferner eine Spule (214) aufweist, die außerhalb der Schlitzröhre angeordnet ist, und die distale Kappe einen Außendurchmesser hat, der kleiner als ein Außendurchmesser der Spule ist.

7. Medizinprodukt nach Anspruch 6, wobei der proximale Schaft (234) einen distalen Bereich (238) mit einer darin gebildeten Tasche (240) aufweist und sich die Schlitzröhre proximal in die Tasche erstreckt und optional die Tasche einen Schulterbereich (244) distal von einem Boden (242) der Tasche aufweist, wobei sich die Spule zum Schulterbereich erstreckt.

8. Medizinprodukt nach Anspruch 6, wobei der proximale Schaft einen verengten Abschnitt (254) aufweist, der sich in das distale Teil erstreckt und die Schlitzröhre bildet und optional ein Übergang vom proximalen Schaft zum verengten Abschnitt des proximalen Schafts einen Schulterbereich (258) bildet, wobei die Spule am Schulterbereich abschließt.

9. Medizinprodukt nach einem der Ansprüche 6 bis 8, wobei ein distales Ende der Spule proximal von einem distalen Ende der Schlitzröhre liegt und sich ein proximales Ende der distalen Kappe über dem distalen Ende der Schlitzröhre proximal erstreckt und am distalen Ende der Spule anliegt.

10. Medizinprodukt nach einem der Ansprüche 6 bis 9, ferner mit einem Kragen (276), der die Schlitzröhre am proximalen Schaft befestigt.

11. Medizinprodukt nach Anspruch 1,
wobei das distale Teil eine geschnittene Röhre (448) aufweist.

12. Medizinprodukt nach Anspruch 11, wobei das distale Teil eine spiralgeschnittene Röhre oder eine geschnittene Röhre mit mehreren abgewinkelten Schnitten aufweist.

13. Medizinprodukt nach Anspruch 11 oder 12, ferner mit einem Formgebungsband (346).

14. Medizinprodukt nach einem der Ansprüche 11 bis 13, wobei das distale Teil eine Spule aufweist, die so konfiguriert ist, dass Fluid zwischen einer Position außerhalb der Spule und einer Position innerhalb der Spule fließen kann, und das Medizinprodukt ferner einen Fluidanschluss (354) aufweist, der proximal vom Hohlraum gebildet ist.

## Revendications

1. Dispositif médical pour mesurer la pression sanguine, comprenant :
une tige proximale (18, 234) incluant une lumière proximale (236) s'étendant dans la tige proximale ;
un élément distal (16, 200) définissant une lumière distale (210) s'étendant dans l'élément distal, la lumière distale alignée avec la lumière proximale et incluant une surface interne de lumière distale ;
une coiffe distale (38, 220) fixée à l'élément distal, la coiffe distale incluant une surface interne de coiffe distale (246) définissant un espace vide (40, 224) ;
un capteur de pression optique (44, 226) disposé dans l'espace vide dans la coiffe distale ; et
un câble à fibres optiques (46, 228) couplé au capteur de pression optique et s'étendant de manière proximale dans la lumière distale et la lumière proximale, le câble à fibres optiques ayant une surface externe ;
où une tolérance entre la surface externe du câble à fibres optiques et la surface interne de la surface interne de lumière distale limite le mouvement radial relatif du capteur de pression optique par rapport à la surface interne de coiffe distale de sorte que le capteur de pression optique est empêché d'entrer en contact avec la surface interne de coiffe distale, **caractérisé en ce que** :
la tolérance étant dans la plage d'environ 0,005 mm à environ 0,05 mm.

2. Dispositif médical selon la revendication 1, où un espacement entre le capteur de pression optique et la surface interne de coiffe distale protège encore le capteur de pression optique contre un contact avec la surface interne de coiffe distale.

3. Dispositif médical selon l'une quelconque des revendications 1 ou 2, comprenant en outre une ou plusieurs ouvertures (42, 230, 232) formées dans la coiffe distale pour permettre à un fluide d'entrer dans l'espace vide.

4. Dispositif médical selon l'une quelconque des revendications 1 à 3, où l'élément distal comprend un tube fendu (204).

5. Dispositif médical selon la revendication 4, où la coiffe distale a un diamètre interne qui est sensiblement égal à un diamètre externe du tube fendu.

6. Dispositif médical selon la revendication 5, où l'élément distal comprend en outre une spirale (214) disposée à l'extérieur du tube fendu, et la coiffe distale a un diamètre externe qui est inférieur à un diamètre externe de la spirale.

7. Dispositif médical selon la revendication 6, où la tige proximale (234) inclut une région distale (238) ayant un évidement (240) formé à l'intérieur et le tube fendu s'étend de manière proximale dans l'évidement et éventuellement l'évidement inclut une région d'épaulement (244) distale par rapport à un fond (242) de l'évidement, la spirale s'étendant jusqu'à la région d'épaulement.

8. Dispositif médical selon la revendication 6, où la tige proximale inclut une partie rétrécie (254) qui s'étend dans l'élément distal et forme le tube fendu et éventuellement une transition de la tige proximale à la partie rétrécie de la tige proximale forme une région d'épaulement (258), la spirale se terminant au niveau de la région d'épaulement.

9. Dispositif médical selon l'une quelconque des revendications 6 à 8, où une extrémité distale de la spirale est située de manière proximale par rapport à une extrémité distale du tube fendu et une extrémité proximale de la coiffe distale s'étend de manière proximale au-dessus de l'extrémité distale du tube fendu et bute contre l'extrémité distale de la spirale.

10. Dispositif médical selon l'une quelconque des revendications 6 à 9, comprenant en outre une bague (276) fixant le tube fendu à la tige proximale.

11. Dispositif médical selon la revendication 1, où l'élément distal comprend un tube coupé (448).

12. Dispositif médical selon la revendication 11, où l'élément distal comprend un tube coupé en spirale ou un tube coupé ayant une pluralité de coupures en biais.

13. Dispositif médical selon l'une quelconque des revendications 11 ou 12, comprenant en outre un ruban de mise en forme (346).

14. Dispositif médical selon l'une quelconque des revendications 11 à 13, où l'élément distal comprend une spirale qui est configurée pour permettre à un fluide de s'écouler entre une position extérieure à la spirale et une position intérieure à la spirale et le dispositif médical comprend en outre un orifice à fluide (354) défini de manière proximale par rapport à l'espace vide.
